# EUROPÄISCHE PATENTSCHRIFT

(11) **EP 3 911 661 B1**
(45) Veröffentlichungstag und Bekanntmachung des Hinweises auf die Patenterteilung: **01.05.2024**
(21) Anmeldenummer: 20703724.3
(22) Anmeldetag: 04.02.2020
(51) Int. Cl.: C07K 1/04

(54) **ULTRASCHALL-GESTÜTZTES VERFAHREN ZUR FESTPHASENPEPTIDSYNTHESE UND VORRICHTUNG**
ULTRASOUND ASSISTED PROCESS FOR SOLID-PHASE PEPTIDE SYNTHESIS AND DEVICE
PROCÉDÉ DE SYNTHÈSE DE PEPTIDES EN PHASE SOLIDE ET DISPOSITIF SUPPORTÉ PAR ULTRASON

(30) Priorität: 15.01.2019 DE 102019100924
(43) Veröffentlichungstag der Anmeldung: 24.11.2021
(73) Patentinhaber: Kreuzer, Oliver Johannes, 13469 Berlin (DE)
(72) Erfinder: Kreuzer, Oliver Johannes, 13469 Berlin (DE)
(74) Vertreter: ETL IP Patent- und Rechtsanwaltsgesellschaft mbH
(86) Internationale Anmeldenummer: PCT/EP2020/052681
(87) Internationale Veröffentlichungsnummer: WO 2020/148463

(56) Entgegenhaltungen:
- CN-U- 203 569 010
- CN-U- 209 052 610
- DE-A1- 4 006 349
- US-A- 3 867 269
- US-A1- 2012 132 572
- US-A1- 2013 310 265
- US-B1- 6 277 332
- WOLCZANSKI GRZEGORZ ET AL: "A faster solid phase peptide synthesis method using ultrasonic agitation", TETRAHEDRON LETTERS, Bd. 60, Nr. 28, 1. Juni 2019 (2019-06-01), Seiten 1814-1818, XP085725603, ISSN: 0040-4039, DOI: 10.1016/J.TETLET.2019.05.069
- PEREZ J MANUEL ET AL: "The use of power ultrasound coupled with magnetic separation for the solid phase synthesis of compound libraries", BIOORGANIC & MEDICINAL CHEMISTRY LETTERS, PERGAMON, AMSTERDAM, NL, Bd. 10, Nr. 2, 11. Mai 2017 (2017-05-11), Seiten 171-174, XP085015733, ISSN: 0960-894X, DOI: 10.1016/S0960-894X(99)00651-4
- FRANCESCO MERLINO ET AL: "Boosting Fmoc Solid-Phase Peptide Synthesis by Ultrasonication", ORGANIC LETTERS, Bd. 21, Nr. 16, 30. Juli 2019 (2019-07-30) , Seiten 6378-6382, XP055693353, ISSN: 1523-7060, DOI: 10.1021/acs.orglett.9b02283

## Beschreibung

Die Erfindung betrifft ein Verfahren zur Durchführung einer Festphasenpeptidsynthese, eine automatisierte parallele Festphasenpeptidsynthese sowie eine Vorrichtung eingerichtet ein solches Verfahren durchzuführen.

Die Festphasen-Peptidsynthese, (SPPS für engl. solid phase peptide synthesis oder auch Merrifield-Synthese) ist eine 1962 vom Nobelpreisträger Robert Bruce Merrifield eingeführte Peptidsynthese, bei der ein unlöslicher polymerer Träger verwendet wird. Ein lineares Peptid wird durch schrittweises Anknüpfen der sequenzspezifischen, temporär geschützten Aminosäuren aufgebaut, wobei das C-terminale Ende der wachsenden Polypeptidkette kovalent mit einem Kunstharzträger verknüpft ist. Zur Gewährleistung einer kontrollierten Reaktionsführung und zur Vermeidung von Nebenreaktionen müssen reaktive funktionelle Seitenketten der Aminosäuren durch geeignete Schutzgruppen blockiert werden. Während die α-Aminogruppe der zu verknüpfenden Aminosäure nur bei der eigentlichen Kupplungsreaktion geschützt sein muss, werden permanente Seitenkettenschutzgruppen erst nach Beendigung der Synthese vom Peptid abgespalten. Im Gegensatz zur ribosomalen Proteinbiosynthese erfolgt die Verlängerung der Peptidkette vom C- zum N-Terminus. Als polymerer Träger hat sich ein Copolymerisat aus Polystyrol und 1-2% 1,4-Divinylbenzol bewährt. Die durch Perlpolymerisation erhaltenen Harzkügelchen mit einem Durchmesser zwischen 20 und 100 µm quellen in den für die Synthese verwendeten Lösungsmitteln und werden dadurch für die Reagenzien permeabel. Als intermediäre α-Aminoschutzgruppen werden hauptsächlich die tert.-Butyloxycarbonyl (Boc)- und die Fluorenyl-9-methoxycarbonyl (Fmoc)-Gruppe eingesetzt. Die Boc-Gruppe ist stabil gegenüber katalytischer Hydrierung und alkalischer Hydrolyse und lässt sich durch milde Acidolyse, z. B. mit 50% Trifluoressigsäure (TFA) abspalten. Die ständige Wiederholung der sauren Deblockierungsreaktionen nach den einzelnen Kupplungsschritten kann zur partiellen Deblockierung von Seitenkettenschutzgruppen sowie zu einer geringen Hydrolyse der Ankerbindung zum polymeren Träger führen. Die Fmoc-Gruppe besitzt den Vorteil, dass sie durch Behandlung mit geeigneten Basen, wie Morpholin, 2-Aminoethanol oder Piperidin, abgespalten werden kann. Werden als Ankergruppierung am polymeren Träger sowie zum Schutz der Drittfunktionen entsprechender Aminosäurebausteine säurelabile, gegen Basen resistente Gruppierungen verwendet, dann können vorteilhafterweise intermediäre und permanente Schutzgruppen unabhängig voneinander abgespalten werden.

Die Kupplungsreaktion (auch Kondensation oder Peptidpropagation) ist ein äußerst wichtiger Schritt für die Synthese, weil ein vollständiger Umsatz die Grundvoraussetzung für die Einheitlichkeit des Endprodukts ist. In der Regel wird das Reagens im Überschuss eingesetzt, wobei Anhydride, Aktivester oder sog. in-situ-Aktivatoren, bei denen intermediär aktivierte Esterderivate entstehen, bevorzugte Verwendung finden. Die sich ständig wiederholenden Reaktionsschritte Abspaltung der α-Aminoschutzgruppe und Anknüpfung der nächsten Nα-geschützten Aminosäure (Kupplungsreaktion, Kondensation) haben die weitgehende Automatisierung der Syntheseschritte und Konstruktion von Peptidsynthesizern ermöglicht, von denen die meisten nach dem Durchflussprinzip arbeiten. Das Harz befindet sich dabei in einer Säule mit einer Fritte am Boden, so dass Reagenzien und Lösungsmittel automatisch zugeführt, mit dem Trägermaterial vermischt und anschließend abgesaugt werden können. Die Wiederholung der Schritte erfolgt bis zur gewünschten Länge des aufzubauenden Peptids. Abschließend erfolgt die Abspaltung des synthetisierten Peptids vom polymeren Träger. Die Ablösung von der Harzmatrix gelingt mittels Reagenzien, die in Abhängigkeit vom gewählten Schutzgruppenschema die Ankerbindung zwischen C-terminaler Aminosäure und Träger selektiv spalten, oder auch synchron eine partielle oder vollständige Deblockierung des synthetisierten Peptids bewirken. Aus der Festphasenpeptidsynthese entwickelte sich die multiple Peptidsynthese. [E. Atherton u. R.C. Sheppard Solid-Phase Synthesis - A Practical Approach, Oxford University Press, 1989; H.-D. Jakubke Peptide: Chemie und Biologie, Spektrum Akademischer Verlag Heidelberg, 1996]

Eine große Herausforderung der automatisierten Peptidsynthese ist es Kreuzkontaminationen zu vermeiden, da bei bekannten Verfahren und Automaten die Reagenzien durch gleiche Schlauchsysteme und Kanülen geleitet werden. Um Kreuzkontaminationen zu verhindern, wird bei bekannten Geräten das gesamte System mit großen Volumina an Spülmitteln gespült.

Die DE 101 31 088 B4 setzt hier an und stellt eine Vorrichtung bereit, die eine automatisierte, zeitgleiche, multiple und parallele Synthese ermöglicht, bei der die Kreuzkontaminationen ausgeschlossen werden können. Dies führt zu einer deutlichen Reduktion von Synthesezeit.

Ein anderer Ansatz die Synthesezeit bei der Peptidsynthese zu reduzieren, ist die Einwirkung von Mikrowellenstrahlung auf die Reagentien während der Synthese. Dies ermöglicht eine Reduktion der Synthesezeit auf ein Zehntel. Allerdings müssen mikrowellenunterstützte Reaktionen in speziellen Schutzräumen durchgeführt werden. Die Methode ist daher auf kleinere Reaktoren und damit geringe Durchsatzmengen begrenzt. Zudem zeigte sich, dass nicht alle der üblichen Schutzgruppen gegenüber Mikrowellenstrahlung stabil sind, so dass es zu verringerten Ausbeuten und mehr Verunreinigungen kommen kann.

1977 wurde in CA 101 93 24 der Versuch publiziert, die Synthese mittels Ultraschall zu unterstützen. Es zeigte sich jedoch in den Folgejahren, dass diese Methode, zumindest in der gezeigten Form nicht zu den gewünschten Ergebnissen führte. Es konnte bei üblichen Verfahren keine reproduzierbare Beschleunigung der Synthesezeit festgestellt werden.

Die Dokumente CN 203 569 010 U und US 6 277 332 B1 offenbaren eine Vorrichtung zur Durchführung einer Festphasenpeptidsynthese/ parallelen Festphasenpeptidsynthese sowie ein Verfahren zur Durchführung einer Festphasenpeptidsynthese an einem Trägermaterial, wobei die Synthese in einem flüssigen Reaktionsmedium stattfindet und zumindest während einer der Schritte zumindest zeitweise Ultraschall mit einer Frequenz von 40 kHz auf das Reaktionsmedium einwirkt.

Aufgabe der Erfindung ist es nun, die Synthesezeit einer Festphasenpeptidsynthese bei gleichbleibender oder besserer Ausbeute und Reinheit weiter zu beschleunigen. Das Verfahren soll insbesondere auf automatisierte, parallele Verfahren anwendbar sein.

Diese Aufgabe wird durch ein Verfahren zur Durchführung einer automatisierten parallelen Festphasenpeptidsynthese sowie einer Vorrichtung zu deren Durchführung mit den Merkmalen der unabhängigen Ansprüche gelöst.

Somit betrifft ein erster Aspekt der Erfindung ein Verfahren zur Durchführung einer Festphasenpeptidsynthese (im Weiteren auch Synthese oder Peptidsynthese). Das erfindungsgemäße Verfahren umfasst die Schritte
a) Binden einer am N-Terminus mit einer Schutzgruppe geschützten Aminosäure über einen C-Terminus der Aminosäure an ein festes Trägermaterial,
b) Abspalten der Schutzgruppe,
c) Durchführen zumindest einer Peptidpropagation, sowie
d) Abbrechen der Reaktion durch Abspalten des Peptids vom Trägermaterial,
wobei die Schritte a) bis d) in einem flüssigen Reaktionsmedium stattfinden und zumindest während einer der Schritte zumindest zeitweise Ultraschall mit einer Frequenz im Bereich von >100 bis 2000 kHz auf das Reaktionsmedium wirkt.

Es zeigte sich, dass Ultraschall erst ab einer Frequenz von mehr als 40 kHz einen beschleunigenden Effekt auf die in Rede stehenden Reaktionen bei der Festphasenpeptidsynthese hat. Das erfindungsgemäße Verfahren ermöglicht damit eine reproduzierbare Reduktion der Synthesezeit bei der Festphasenpeptidsynthese, die zumindest im Bereich von durch Mikrowellen unterstützter Peptidsynthese liegt. Vorteilhafterweise sind dabei jedoch keine besonderen Schutzvorkehrungen zu treffen. Ferner sind die notwendigen Geräte kostengünstiger in Beschaffung und Wartung. Damit ist das Verfahren bei nahezu jedem und insbesondere bei parallelen und/oder automatisierbaren Synthesesetups anwendbar.

Unter Schritt a) wird vorliegend verstanden, dass eine funktionelle Gruppe mittelbar oder unmittelbar an ein geeignetes Trägermaterial, beispielsweise ein vorbeladenes oder nicht vorbeladenes Harz beziehungsweise ein Amidharz für die Festphasenpeptidsynthese gebunden wird. Diese funktionelle Gruppe kann insbesondere vermittels eine Fmoc-Schutzgruppe geschützt sein. Hierbei bezieht sich vorbeladen oder nicht vorbeladen darauf, dass bereits zumindest eine erste und optional zumindest eine Folgende, also die in der zu synthetisierenden Aminosäuresequenz primäre Aminosäuren unmittelbar an das Trägermaterial gebunden ist.

Besonders geeignet zeigten sich Frequenzen von mehr als 100kHz, da mit höherer Frequenz eine deutlichere Synthesezeitreduktion erzielt werden kann. Es zeigte sich, dass die Bildung von Kavitäten für den positiven Effekt auf die Peptidsynthese, insbesondere die Qualitätssteigerung erheblich ist. Die damit verbundene Kavitation setzt ab einer Frequenz von 40kHz ein und verstärkt sich mit zunehmender Frequenz. Im Frequenzbereich von 20 bis 40 kHz finden lediglich Schwingungsanregungen statt. Vorzugsweise überschreiten die Ultraschallfrequenzen des erfindungsgemäßen Verfahrens 2 MHz, insbesondere 1 MHz nicht. Weitere Ausführungen zu bevorzugten Frequenzen folgen.

Die hier neu beschriebene Ultraschall-gestützte Festphasen Peptidsynthese (USPS) gehört in der Chemischen Synthese in die Kategorie der Sonochemie.

Der chemische Effekt des Ultraschalls kann kein direkter Effekt des Schallfeldes sein, da die üblichen Frequenzen um mehrere Größenordnungen zu niedrig sind, selbst eine simple Rotationsbewegung anzuregen.

Es wird vermutet, dass der positive Effekt in direktem Zusammenhang zur durch Ultraschall ausgelösten Kavitation und den damit erzeugten Druckimpulsen zu sehen ist. Kavitationen entstehen in einem Frequenzbereich von 40 kHz bis 2 MHz.

Es sind drei Typen sonochemischer Reaktionen postuliert.
1. In homogenen Systemen durch radikalische oder radikal-ionische Zwischenstufen. In der Kavitationsblase entstehen in wässriger Phase durch extremen Druck und hohe Temperaturen zum Beispiel OH· und H· Radikale, die unter anderem die Darstellung von H₂O₂ in der Blase bewirken.
2. In heterogenen Systemen durch ionische Reaktionen. Diese werden vor allem durch die mechanischen Effekte der Kavitation im Lösungsmittel unterstützt. An festen Partikeln bilden sich asymmetrische Blasen aus. Durch das Platzen der asymmetrischen Blasen an den Partikeln entstehen Flüssigkeitsjets, die in Richtung der einseitig zerplatzenden Blase schießen. Dadurch wird die Aufnahme von Lösungsmittel und gelösten Substanzen in das poröse Material unterstützt. An anderen flüssigen Phasen findet hingegen eine Durchmischung der Phasen statt:
3. In heterogenen Systemen, wo auch radikalische Reaktionen stattfinden können. Dabei kann es sein, dass der radikalische Weg andere Produkte hervorbringt als der ionische, wie beispielsweise bei der Kornblum-Russell Reaktion.

Dabei entstehen mit höherer Wahrscheinlichkeit in niedrigeren Frequenzbereichen Kavitationsblasen, welche dann auch größer werden und asymmetrisch ausgebildet sind. Dadurch findet eine stärkere aber ungleichmäßigere Durchmischung statt. Bei höheren Frequenzen entstehen hingegen mehr kleinere, symmetrische Blasen und es findet vermehrt ein Radikalaustausch zwischen den Kavitationsblasen und der Umgebung statt.

Kavitation ist "die Formation, das Wachstum und der implosive Kollaps der Blasen in einer Flüssigkeit. Der Kavitationskollaps verursacht lokal hohe Temperaturen (~5000 K), hohe Drücke (~1000 atm), enorme Aufheiz- und Abkühlgeschwindigkeiten (>109 K/sec)" und Flüssigkeitsstrahlen (~400 km/h). Kavitationsblasen sind Vakuumblasen (Suslick 1998). Das Vakuum entsteht durch eine sich schnell bewegende Oberfläche und eine reaktionsträge Flüssigkeit. Die daraus resultierenden Druckunterschiede überwinden die Kohäsions- und Adhäsionskräfte innerhalb der Flüssigkeit.

Ab einer Frequenz von 110 kHz und mehr, insbesondere ab 125kHz, vorzugsweise bei 130 kHz und mehr zeigt sich neben einem beschleunigten Reaktionsverlauf und damit verbundener verkürzter Reaktionszeit auch eine Verbesserung der Ausbeute. Es zeigte sich, dass im Vergleich zu Standardsystemen nicht mit einem 40fachen Überschuss an Aminosäure gearbeitet werden muss, sondern die gleichen Ergebnisse bereits mit einem 4fachen Überschuss erzielt werden. Dies führt wiederum zu deutlich reduzierten Reaktandenmengen und mithin zu einer erheblichen Kostenersparnis. Bei Frequenzen im Bereich von 110 bis 500 kHz wurde zudem keine Racemisierung festgestellt, was zu hohen Ausbeuten führt.

In bevorzugter Ausgestaltung des erfindungsgemäßen Verfahrens ist vorgesehen, dass der Ultraschall über ein externes flüssiges Bad auf das Reaktionsmedium übertragen wird. Dies ist ein deutlicher Unterschied zu Mitteln, die den Ultraschall direkt oder ausschließlich über feste Übertragungsmittel auf das Reaktionsmedium übertragen. Es zeigte sich, dass die Übertragung über zumindest ein flüssiges Medium gleichmäßigere, reproduzierbarere und schonendere Ergebnisse der Synthese liefert. Die notwendige Synthesezeit zeigte bei Verwendung eines eine Flüssigkeit aufweisenden Übertragungsmittels eine geringere Streuung als beispielsweise die Synthesezeit unter Verwendung einer in das Reaktionsmedium eingetauchten Sonde. Darüber hinaus ist der Versuchsaufbau deutlich einfacher als bei der Verwendung einer Sonde. Das Eintauchen einer Sonde würde unweigerlich zu Kontaminationen der Sonde führen und ein regelmäßiges Reinigen der Sonde erfordern, was den Gewinn an Synthesezeit egalisieren oder zumindest deutlich schmälern würde.

Der Energieanteil, der in Kavitation umgewandelt wird, hängt von mehreren Faktoren ab, die die Bewegung angeben, die von den kavitationserzeugenden Geräten auf die Flüssigkeit übertragen werden. Die Intensität der Beschleunigung ist einer der wichtigsten Faktoren, der die effiziente Umwandlung von Energie in Kavitation beeinflusst. Höhere Beschleunigung erzeugt höhere Druckunterschiede. Dadurch erhöht sich die Wahrscheinlichkeit, dass Vakuumblasen anstatt der Wellen in der Flüssigkeit erzeugt werden. Dies bedeutet: Je höher die Beschleunigung, desto höher ist der Energieanteil, der in Kavitation umgewandelt wird. Bei einem (Ultra-)Schallwandler wird die Intensität der Beschleunigung durch die Schwingungsamplitude festgelegt. Neben der Intensität des Ultraschalls ist es auch wichtig, dass die Flüssigkeit so beschleunigt wird, dass möglichst geringe Verluste aus den Turbulenzen, der Reibung und der Wellengeneration entstehen. Dazu ist der Weg der unilateralen Bewegungsrichtung am besten geeignet.

Daher ist die Wahl des Übertragungsmediums für den Effekt in der Peptidsynthese entscheidend. Neben der Wahl des Zustands ist vor allem auch der Stoff des Übertragungsmediums zu optimieren. Neben Wasser als Übertragungsmedium werden bevorzugt auch organische Lösungsmittel, insbesondere niedere und mittlere Alkohole, wie Ethanol, Propanol und Butanol, als Übertragungsmedium eingesetzt.

Mit Vorteil werden verschiedene Übertragungsmedien in Kombination gewählt. Dabei ist der Versuchsaufbau als Bad-im-Bad zu verstehen. Mit anderen Worten, es findet die Reaktion in einem Reaktionsmedium statt, welches in einem (Reaktions-)Gefäß angeordnet ist. Dieses Reaktionsgefäß ist wiederum in einem Gefäß mit einem ersten Übertragungsmedium angeordnet, welches seinerseits in einem weiteren Übertragungsmedium angeordnet ist. Der Ultraschall wird demnach über das weitere Übertragungsmedium auf das erste Übertragungsmedium und von dort auf das Reaktionsmedium übertragen.

Mit besonderem Vorteil handelt es sich bei dem ersten Übertragungsmedium um niedere und mittlere Alkohole, insbesondere der vorgenannten Art und/oder bei dem weiteren Übertragungsmedium um Wasser.

Mit hohen Ultraschallfrequenzen steigt die Temperatur im flüssigen Bad erwartungsgemäß an. Bei Frequenzen bis 500kHz lässt sich dieser Effekt jedoch sehr gut kontrollieren, da die dabei auftretende Temperaturerhöhung beispielsweise leicht mit einer Kühleinrichtung ausgeglichen werden kann wie z.B. die Kühlung des Wasserbades vermittels Durchlaufkühler (Kryostat) oder Peltierelemente so dass auch hier potentiell auftretende Racemisierung keine Beeinträchtigung der Ausbeute darstellt.

Bei der Anwendung von Frequenzen deutlich über 500 kHz bis zu 1000 kHz oder darüber, zeigte sich, dass es zur Qualitätssicherung sinnvoll ist, der Temperaturerhöhung, beispielsweise durch Kühlung des Bads entgegenzuwirken.

Das Ultraschallbad wird vorzugsweise einer Temperaturregelung unterzogen und zwar auf einen Temperaturbereich von 20 bis 100 °C, vorzugsweise von 20 bis 70 °C, besonders bevorzugt von 40 bis 50 °C.

In weiter bevorzugter Ausgestaltung der Erfindung ist vorgesehen, dass die Aminosäure am N-Terminus durch eine basenlabile, insbesondere eine mittels sekundären Aminen abspaltbare, temporäre (primäre) Schutzgruppe, insbesondere Fluorenylmethoxycarbonyl (Fmoc), geschützt ist. Diese Schutzgruppen zeigten sich im Vergleich zu den bei boc-Verfahren verwendeten Schutzgruppen besonders stabil gegenüber Ultraschall in den erfindungsgemäßen Frequenzen, so dass besonders hohe Ausbeuten mit hoher Reinheit erzielt werden konnten. Das Entschützen erfolgt vorzugsweise mittels einer geeigneten Base, wie voranstehend oder bei den Versuchsergebnissen beschrieben. Bevorzugt wird 20% Piperidin in DMF (Dimethylformamid) verwendet.

Bislang konnte der Vorteil einer ultraschall unterstützten Peptidsynthese lediglich für eine BocOC- Synthese bestätigt werden. Es wurde sogar gezeigt, dass bei Fmoc-basierter Synthese eine Ultraschallunterstützung nicht angezeigt ist, da die verwendeten Harze unter Ultraschall pulverisieren. Aufgrund der unterschiedlichen Reaktionsmedien und verwendeten Schutzgruppen der Seitenketten wurde davon ausgegangen, dass lediglich die Kopplung ans Harz von Ultraschall unterstützt werden kann. Überraschenderweise konnte jedoch mit vorliegendem Verfahren in den beschriebenen Frequenzbereichen auch für Fmoc-basierte Peptidsynthesen ein Vorteil bezogen auf Reaktionszeit und Ausbeute durch Ultraschallunterstützung während der einzelnen Syntheseschritte gezeigt werden.

Reaktive Seitenketten der mittels des erfindungsgemäßen Verfahrens zu synthetisierenden Peptide sind bevorzugt ebenfalls durch (sekundäre) Schutzgruppen geschützt. Hierbei erwiesen sich in Abhängigkeit der zu schützenden funktionellen Gruppen säurestabile Schutzgruppen, insbesondere auszuwählen aus der Gruppe S-2,4,6-Trimethoxybenzyl (Tmob), Triphenylmethl (Trt), tert-Butyl (tBu), tert-Butyloxycarbonyl (Boc), 2,2,4,6,7-Pentamethyldihydrobenzofuran-5-sulfonyl (Pbf) als besonders stabil bei der erfindungsgemäßen Peptidsynthese mittels Ultraschall im erwähnten Frequenzbereich. Bezogen auf das erfindungsgemäße Verfahren sind beispielsweise Fmoc-Aminosäuren ausgewählt aus der folgenden die jeweilige Aminosäure als Single-letter-code oder Threeletter-code notierten Gruppe zur Peptidpropagation besonders geeignet: Fmoc-A-OH, Fmoc-C(Trt)-OH, Fmoc-D(OtBu)-OH, Fmoc-E(OtBu)-OH, Fmoc-F-OH, Fmoc-G-OH, Fmoc-H(Trt)-OH, Fmoc-I-OH, Fmoc-K(Boc)-OH, Fmoc-L-OH, Fmoc-M-OH, F moc-N(Trt)-OH, Fmoc-P-OH, Fmoc-Q-Trt-OH, Fmoc-R-Pbf-OH, Fmoc-S-tBu-OH, Fmoc-T-tBu-OH, Fmoc-V-OH, Fmoc-W(Boc)-OH, Fmoc-Y-(tBu)-OH, Fmoc-Gln(Tmob)-OH, Fmoc-Asn(Tmob)-OH Hiemit sind sowohl die L-Formen als auch die D-Formen der Aminosäuren gemeint. Darüber hinaus lassen sich auch spezielle Seitenkettengeschützte posttranslational modifizierte Aminosäuren verwenden wie z.B.
Für die Phosphorylierung von Ser/Thr :
   Fmoc-Ser(PO(OBzl)OH), Fmoc-Thr(PO(OBzl)OH), Fmoc-Tyr(PO(OMe)₂), Fmoc-Tyr(PO(OBzl)OH), Fmoc-Tyr(PO(OBzl)₂)-OH, Fmoc-Tyr(PO₃H₂)-OH, Fmoc-Tyr(PO(NMe₂)₂), Fmoc-Tyr(PO(NMe₂)₂), Fmoc-Ppa(Bzl)-OH, Fmoc-Pmp-OH, Fmoc-F₂Pmp-OH,
Für die Sulfatierung von Tyr:
   Fmoc-Tyr(SO₃nP)-OH, Fmoc-Tyr(SO₃DCV)-OH,
Für die Methylierung von Arg:
   Fmoc-Arg(Me,Pbf)-OH, Fmoc-ADMA(Pbf)-OH, Fmoc-SDMA(Boc₂)-ONa,
Für die Methylierung von Lys:
   Fmoc-Lys(Me,Boc)-OH, Fmoc-Lys(Me₂)-OH, Fmoc-Lys(Me₃Cl)-OH
Für die Citrullinierung:
   Fmoc-Citrulline-OH
Für die Glykosylierung von Asn:
   Fmoc-Asn(β-DGIcNAc(Ac)₃)-OH, Fmoc-Asn(β-DGIcNAc(Ac)₃-(1-4)-β-DGIcNAc(Ac)₂)-OH
Für die Glykosylierung von Ser/ Thr:
   Fmoc-Ser/Thr(α-DGlnNAc(Ac)₃)-OH, Fmoc-Ser/Thr(β-DGal(Ac)₄-(1-3) α-DGlnNAc(Ac)₂)-OH,
   Fmoc-Ser/Thr(sialylOMe(Ac)₄-(1-6)-α-D-GlnNAc(Ac)₂)-OH, Fmoc-Ser/Thr(sialylOMe(Ac)₄-(1-3)-β-D-Gal(Ac)₃-(1-3) α-DGlnNAc(Ac)₂)-OH
Ebenso können für die Synthese schwieriger Peptidsequenzen entwickelt Synthesebaustein zur Anwendung kommen, wie FmocPseudoProline Dipeptide, sogenannte Dmb Bausteine: wie Fmoc-(Dmb)Gly-OH, oder als Dipeptid FmocXaaDmbGly, Hmb-Bausteine:
   FmocHmbXaa, sowie Hmsb-Bausteine, Hnb-Bausteine, Mmsb- Bausteine, nicht natürliche Aminosären wie:
Naphthylalanine, Fmoc-L-2Nal-OH
Ornithine, Fmoc-L-Orn(Aloc)-OH sowie die methylierten Varianten
Polyethylen Glykole, Fmoc-O1Pen-OH, Fmoc-AEEP, Fmoc-TTDS-OH und alle weiteren Fmoc - geschützten Amino Polyethylen Glykol Säuren.
Besonders derivatisierte Aminosäuren wie z.B.:
   Fmoc-Lys(biotin)-OH, FMOC-Lys(Cy₅)-OH

Generell lassen sich in der USPS alle Bausteinen einsetzen, die über eine temporär geschützte Amin-Funktion, bevorzugt Fmoc-geschützt und eine in einen Aktivester überführbare Carbonsäure Funktion bzw. über eine Amin-reaktive Gruppe verfügen.

Mit Vorteil wirkt Ultraschall in genau einem Schritt, insbesondere in Schritt c) auf das Reaktionsmedium ein. Alternativ oder zusätzlich wirkt Ultraschall in zumindest einem weiteren Schritt, vorzugsweise Schritt a), b) und/oder Schritt d) auf das Reaktionsmedium. Die reaktionsbeschleunigenden Eigenschaften des erfindungsgemäßen Ultraschalls sind in jedem der genannten Schritte zu beobachten.

Hierbei ist besonders bevorzugt, dass das Einwirken des Ultraschalls zwischen oder in den einzelnen Schritten nicht unterbrochen wird, da eine Unterbrechung zu reduzierter Ausbeute führen kann.

Die bislang größte Reduktion der Synthesezeit bei hohen Ausbeuten wurde erzielt, wenn während der gesamten Synthese, also auch beim Waschen, beim Entschützen und beim Kondensieren beziehungsweise Koppeln Ultraschall in den genannten Bereichen auf das Reaktionsmedium einwirkte. Beim Vorquellen als vorbereitenden Schritt hingegen ist Ultraschall nicht zwingend vorteilhaft, ebenso wenig beim abschließenden Waschen.

Die Festphasenpeptidsynthese umfasst mehrere voneinander zu unterscheidende Waschschritte. Die einzelnen Typen von Waschschritten sind anhand ihrer jeweils vorgeschalteten Reaktionen zu unterscheiden. So sind zumindest ein Waschen nach dem Koppeln der ersten Aminosäure an das Harz (Initiales Waschen), ein Waschen jeweils nach dem Schritt b), dem Entkoppeln der Schutzgruppe (im Weiteren Schritt W_{b}), einem Waschen nach dem Koppeln einer Aminosäure zur Verlängerung der Peptidkette (im Weiteren Schritt W_{c}) und einem abschließenden Waschen nach Schritt d), dem Abspalten der letzten temporären Schutzgruppe fertigen Peptids vom Trägermaterial (im Weiteren Schritt W_{d}) zu nennen.

Während der einzelnen Waschschritte wirkt bevorzugt ebenfalls Ultraschall auf die Reaktion ein und bewirkt eine deutliche Reduktion notwendigen Spülmittels und Spülzeit. So erzielt das Waschen mit nur einem Spülschritt mit Ultraschall bereits die gleichen Ergebnisse wie das üblicherweise viermalige Spülen bei der Standardsynthese. Insbesondere von Bedeutung für eine Ausbeuteerhöhung und Qualitätssteigerung ist der Waschschritt W_{c}, also das Waschen nach Schritt c). Untersuchungen haben gezeigt, dass auf den Waschschritt W_{b} sogar vollständig verzichtet werden kann. Bevorzugt werden jedoch alle Waschritte im erfindungsgemäßen Verfahren durchgeführt.

Bevorzugt, insbesondere für diesen Waschschritt sind Ultraschallfrequenzen im Bereich von 100 bis 500 kHz, vorzugsweise im Bereich von 100 bis 200 kHz, insbesondere im Bereich von 120 bis 140 kHz. In diesen Bereichen konnte die Menge des zum Waschen beziehungsweise Spülen notwendigen Lösungsmittels, beispielsweise DMF, sowohl innerhalb der einzelnen Spülgänge eines Waschschritts, derart reduziert werden, dass nur noch ein einziger Spülschritt pro Waschschritt W_{b} notwendig ist.

Mit besonderem Vorteil ist vorgesehen, dass Ultraschall in den oben angegebenen Frequenzbereichen während aller Schritte der Peptidsynthese a) bis d) inklusive der Waschschritte W_{b}, W_{c} und W_{d}, insbesondere ohne vollständige Unterbrechung auf das Reaktionsmedium einwirkt.

Dabei wurde beobachtet, dass je nach zu synthetisierendem Peptid für die einzelnen Schritte a) bis d) und insbesondere für die Schritt W_{b}, _{c und d} unterschiedliche Frequenzen in Bezug auf Qualitätssteigerung und Reaktionszeiterniedrigung optimal sind, also vorteilhaftere Auswirkungen zeigen. Somit ist bevorzugt, dass in den einzelnen Schritten Ultraschall in unterschiedlichen Frequenzen auf das Reaktionsmedium einwirkt. Insbesondere ist bevorzugt, dass die Frequenz zwischen den Schritten wechselt und/oder Ultraschall mit unterschiedlichen Frequenzen einander überlagern.

Bei dem Trägermaterial handelt es sich um Materialien, die dem Fachmann für Peptidsynthesen grundsätzlich bekannt sind. Dabei handelt es sich um Kunstharze/Syntheseharze, wobei besonders vorteilhaft Harze aus nachfolgender Gruppe sind:
Knorr Amid Harz LS 1%DVB, Wang Harz, Chlortrityl Harz, PRG Harze, Tentagel Harze, Chemmatrix Harze.

Allgemein vorbeladene oder nicht vorbeladene und/oder funktionalisierte Harze (Resin) für die Festphasen Synthese.

Bevorzugt sind Syntheseharze die nicht paramagnetisch sind, da die Trennung der syntehtisierten Peptide mittels Magnetismus deutlich aufwendiger ist, als mittels Filtration und es sich zeigte, dass paramagnetische Harze im Ultraschall bei niedrigen Frequenzen (bis 40 kHz) bis hin zur Bildung feinster Partikel zerkleinert werden und im Zuge dessen die Filtermaterialien verstopfen.

Im Rahmen der Erfindung, vorzugsweise verwendete Lösemittel sind DMF (N,N-Dimethylformamid), NMP (N-Methyl-2-pyrrolidon) oder DMA (N,N-Dimethylacetamid).

Als Base zum Katalysieren der Kondensationsreaktion wird vorzugsweise NMP, 4-Methylmorpholin oder DIPEA, Diisopropylethylamin in DMF oder einem anderen Lösemittel verwendet.

Eine Lösung zum Abspalten der temporären Fmoc-Schutzgruppe ist vorzugsweise 20% Piperidin in DMF. Andere Abspaltmethoden sind dem Fachmann grundsätzlich bekannt.

Vorzugsweise wird als Kopplungsreagenz HBTU (2-(1H-Benzotriazol-1-yl)-1,1,3,3-tetramethyluronium-hexafluorophosphat), HCTU (2-(6-Chlor-1H-benzotriazol-1-yl)-1,1,3,3-tetramethylaminium-hexafluorophosphat), PyBOP (Benzotriazol-1-yl-oxytripyrrolidinophosphonium-hexafluorophosphat), DCC, Dicyclohexylcarbodiimid; DIC, Diisopropylcarbodiimid; oder EDC, 1-Ethyl-3-(3-dimethylaminopropyl)carbodiimid eingesetzt.

Alle Aminosäuren/Reagenzien werden in dem verwendeten Lösemittel gelöst, wodurch das/die Reaktionsmedium/Reaktandenmedium/Reagenzienlösung erhalten wird. Wenn im Rahmen des Verfahrens und der Vorrichtung auf Reagenzien verwiesen wird, wird darunter auch eine Lösung der Reagenzien verstanden.

Die Konzentration der eingesetzten Aminosäure ist immer abhängig vom Synthesemaßstab und deren Löslichkeit im verwendeten Lösemittel, wie beispielsweise DMF, NMP oder DMA.

Um ein gutes Syntheseergebnis zu erhalten, werden die zu koppelnden Aminosäuren (AA) und das Reagenz zur Ausbildung des Aktivesters(Aktivator) bevorzugt jeweils mindestens äquimolar zum Synthesemaßstab eingesetzt. Üblich ist es allerdings, die AA und den Aktivator gemeinsam äquimolar einzusetzen und diese beide im Überschuss zum Synthesemaßstab zu verwenden. Überschüsse bewegen sich von 4fach bis zu hundertfach. Um mit der Festphasenpeptidsynthese nach dem Stand der Technik Qualitäten zu erzielen, die mit dem erfindungsgemäßen Verfahren vergleichbar sind, ist je nach Peptidsequenz mindestens ein 40facher Überschuss erforderlich. Der Grund ist, dass durch die hohen Überschüsse der Edukte die Entstehung des Produkts, nämlich der verlängerten Peptidkette begünstigt ist. Die Konzentration der Aminosäure reicht hierbei in erster Linie an der Löslichkeit der geschützten AA im verwendete Lösemittel und liegt bevorzugt zwischen 0,2M-0,6M.

Im Rahmen der dieser Anmeldung beschriebenen Versuche wurden die Aminosäuren in einer Konzentration von 0,4M eingesetzt.

Wiederum ist es vorteilhaft, dass der Ultraschall zwischen aufeinander folgenden (ultraschallunterstützten) Schritten a)-d) nie vollständig unterbrochen wird, sondern lediglich die Frequenz geändert wird. Mit Vorteil ist vorgesehen, dass das Verfahren halb-/automatisiert und/oder parallel durchgeführt wird. In Kombination von parallelen automatisierten Verfahren mit der erfindungsgemäßen Ultraschallunterstützung ist es möglich, individuelle, also auf ein bestimmtes Individuum abgestimmte Peptide in einem Maßstab und mit einem Durchsatz herzustellen, der die besondere Form der Krebstherapie mit Neoantigenen einer Vielzahl von Patienten zugänglich macht. Neoantigene sind durch Mutation entstandene Veränderungen in Proteinen der Tumorzellen. Sie können mit der Technik des Next-Generation Sequencing (NGS) identifiziert werden. Üblicherweise bewegt sich die Anzahl unterschiedlicher Neoantigene zwischen 100 und 200 Stück. Eine entsprechende Zahl synthetischer Peptide kann diese Neoantigene in vitro nachstellen und zur tumorspezifischen Impfung eines Patienten verwenden. Die Anzahl, die Zusammensetzung und die Aminosäuresequenzen der Neoantigene sind für jeden Patienten individuell. Somit muss auch die Nachbildung der entsprechenden Peptide von Individuum zu Individuum angepasst, mit anderen Worten personalisiert, werden. Um diese vielversprechende Therapie medizinisch überhaupt nutzbar zu machen, müssen in angemessener Zeit Studien an einer Vielzahl von Patienten unter gleichen Bedingungen durchführbar sein. Dies setzt voraus, dass eine Vielzahl personalisierter Antigene in extrem kurzer Zeit zur Verfügung gestellt werden können. Die beschriebene Form der personalisierten Krebstherapie stellt somit hohe Anforderungen an die Geschwindigkeit, die Parallelität und Qualität für die Synthese von Peptiden. Das erfindungsgemäße Verfahren kann diese Anforderungen erfüllen.

Das erfindungsgemäße Verfahren ist insbesondere der großtechnischen Anwendung zugänglich. Unter großtechnischer Anwendung werden vorliegend Maßstäbe von 11-50 I, insbesondere von 100 bis 500 I Ansatzgröße verstanden.

Somit ist ein weiterer Aspekt der Erfindung eine automatisierte parallele Festphasenpetidsynthese umfassend das erfindungsgemäße Verfahren in einer der beschriebenen Ausgestaltungen.

Das erfindungsgemäße Verfahren wird mit besonderem Vorteil bei Raumtemperatur oder moderater Temperaturerhöhung durchgeführt. Besonders bevorzugt sind Reaktionstemperaturen im Bereich von 20 bis 100 °C, vorzugsweise im Bereich von 20 bis 70 °C, insbesondere im Bereich von 40 bis 60 °C. Dabei findet die Temperierung bevorzugt zumindest in Schritt a), b) und/oder d) statt.

Es zeigte sich, dass das erfindungsgemäße Verfahren unter anderem besonders geeignet ist zur großtechnischen Darstellung von Litraglutid und Semaglutid, insbesondere im Maßstab von mehr als 100 g Produkt.

Ein weiterer Aspekt der Erfindung betrifft eine Vorrichtung zur Durchführung einer Festphasenpeptidsynthese die eingerichtet ist, das erfindungsgemäße Verfahren in einer der beschriebenen Ausgestaltungen durchzuführen. Hierzu weist die erfindungsgemäße Vorrichtung einen Ultraschallwandler auf, der vorzugsweise über ein flüssiges Übertragungsmedium Ultraschall einer Frequenz von mehr als 100 kHz bis 2 MHz, vorzugsweise im Bereich von 100 bis 500 kHz auf das Reaktionsmedium überträgt.

Dabei weist die Vorrichtung bevorzugt ein Mittel zur Aufnahme eines oder mehrerer Synthesegefäßes mit zumindest einer Öffnung zum Einfüllen von Reaktandenmedien, insbesondere einer Syntheseplatte im Mikrotiterplatten Format mit 96, 384, 1536 oder 3456 Reaktionskammern, eines Synthesezylinders oder eines Synthesekolbens beziehungsweise Synthesereaktors, sowie ein eine Übertragungsflüssigkeit aufweisendes Ultraschallbad auf, wobei das Synthesegefäß derart im Ultraschallbad anordenbar ist, dass das Synthesegefäß bis zu einer minimalen Höhe mit der Übertragungsflüssigkeit des Ultraschallbades benetzt ist.

Vorteilhafterweise wird unter minimaler Höhe eine Höhe verstanden, die eine Übertragung des Ultraschalls von einem Ultraschallwandler des Ultraschallgeräts auf das Reaktionsmedium derart sicherstellt, dass der Schall nahezu ausschließlich über das flüssige Übertragungsmedium erfolgt. Hierzu entspricht die Höhe der Oberfläche des Übertragungsmediums an einer Außenfläche des Synthesegefäßes mindestens einer halben Höhe, bevorzugt einer Höhe im Bereich von der Hälfte bis zur vollen Höhe, vorzugsweise Dreiviertel bis zur vollen Höhe, eines Meniskus des Reaktionsmediums im Synthesegefäß. Das Synthesegefäß ist bevorzugt als Syntheseplatte, also zum Beispiel als Mikrotiterplatte, insbesondere parallel und nebeneinander angeordneter Synthesezylinder, beispielsweise in Form von Bechergläsern oder Spritzen oder als Synthesekolben, beispielsweise einem Rundkolben oder einem Reaktor (beispielsweise nach Höchst oder Syringe) ausgestaltet. Insbesondere die Ausgestaltung des Synthesegefäßes als Rundkolben oder Reaktor ist für die Verwendung in der großtechnischen halb-/automatisierten Festphasenpeptidsynthese bevorzugt, da hierbei Maßstäbe von 1-50 I im Kolben oder aber 100 bis 500 I im Reaktor bedient werden können.

Die Verwendung von Mikrotiterplatten oder parallel angeordneter Synthesezylinder ist hingegen besonders für die parallele und automatisierte Festphasenpeptidsynthese bevorzugt, wohingegen vorgenannte eher zur großtechnischen Anwendung einzelner Peptide wie beispielsweise Litraglutid und Semaglutid genutzt werden. Durch die Erfindung wird eine Vorrichtung vorgeschlagen, die gänzlich ohne Dilutoren und Schlauchsysteme für die Zuführung und dosierte Abgabe der Synthesebausteine arbeitet. Für die einzelnen Synthesebausteine sind jeweils separate Synthesestifte vorgesehen, die in einer Halterung der Synthesevorrichtung für die Synthese bereitgestellt sind und aus dieser Halterung vom Greifarm der Vorrichtung erfasst und aufgenommen werden, um eine dosierte Menge des Bausteines auf das in einer Reaktionskammer eines Synthesegefäßes, insbesondere einer Syntheseplatte befindliche Trägermaterial abzugeben.

Reagenzienreservoir und Dosiervorrichtung bilden somit eine in sich geschlossene Einheit. Dadurch entfallen sämtliche Spülvorgänge und die damit verbundenen Nachteile, die bisher bei einem Wechsel der Synthesebausteine und der Reagenzienverteilung notwendig waren.

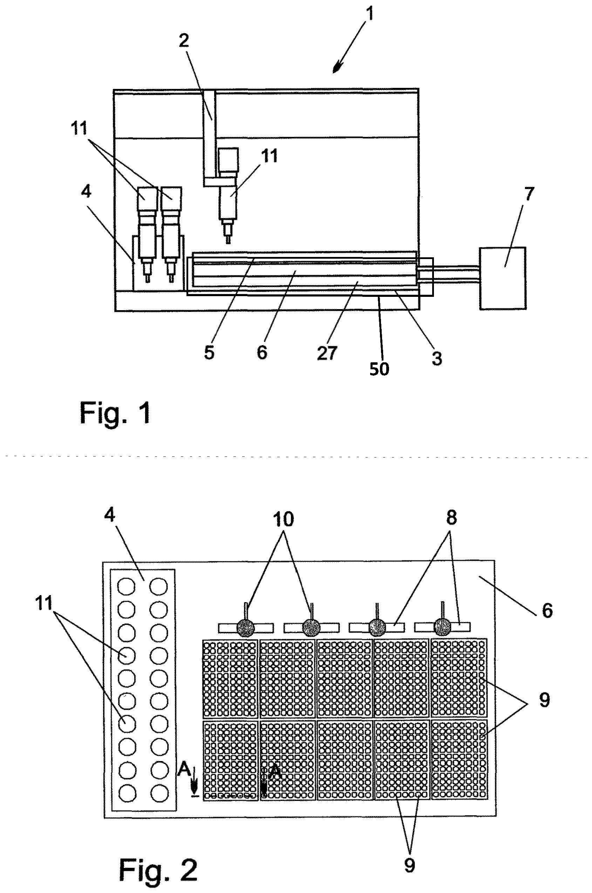

Für die parallele Synthese ist eine Fläche definiert (Fig.2). Der Arbeitsbereich ist so dimensioniert, dass jeder Punkt des Arbeitsbereichs durch den vom Greifarm verfahrenen Synthesestift erreicht werden kann. Auf diesem Arbeitsbereich sind vorzugsweise bis zu 10 Synthesestationen, insbesondere symmetrisch angeordnet. Die Synthesestation ist in ihren Dimensionen vorzugsweise an Standard Mikrotiterplatten angelehnt. Eine Synthesestation kann modular aufgebaut sein und ein Fußteil mit einem Anschluss zur Absaugung von Lösungsmitteln und einem ausgesetzten Rahmen zur Aufnahme der Syntheseplatte umfassen. Je nach Unterteilung der verwendeten Syntheseplatten können beispielsweise parallel 6, 12, 24, 48, 96, 384,1536 oder 3456 individuelle Synthesen in einer Syntheseplatte durchgeführt werden. Für größere Synthesemaßstäbe könne in speziellen Aufnahmen Synthesezylinder bzw. Spritzenkörper verwendet werden.

Nach den weiteren Merkmalen der Erfindung sind die Reaktionskammern der Syntheseplatten öffnungsseitig mit einem permeablen Material, beispielsweise mit einer Fritte verschlossen. Unterhalb des Rahmens können Probenplatten für die Aufnahme der nach Abspaltreaktion gelösten Peptide angeordnet, werden. Mit der vorgeschlagenen Vorrichtung für die Festphasensynthese ist sowohl die Synthese als auch die Abspaltung der gewonnenen Verbindungen vom Trägermaterial, dem Syntheseharz, ohne manuelles Eingreifen möglich.

Die Probenplatten sind mit einzelnen Aufnahmekammern ausgestattet, die in Ihrer Anordnung und Ausbildung mit dem Raster der Reaktionskammern in der Syntheseplatte korrespondieren. Auf diese Weise ist eine fehlerfreie und leichte Zuordnung der jeweiligen Verbindung nach ihrer Abspaltung vom Trägermaterial bzw. Syntheseharz gewährleistet. Eine direkte Übertragung der Reaktionsprodukte auf Hochdurchsatzscreening-Straßen wird so einfach möglich.

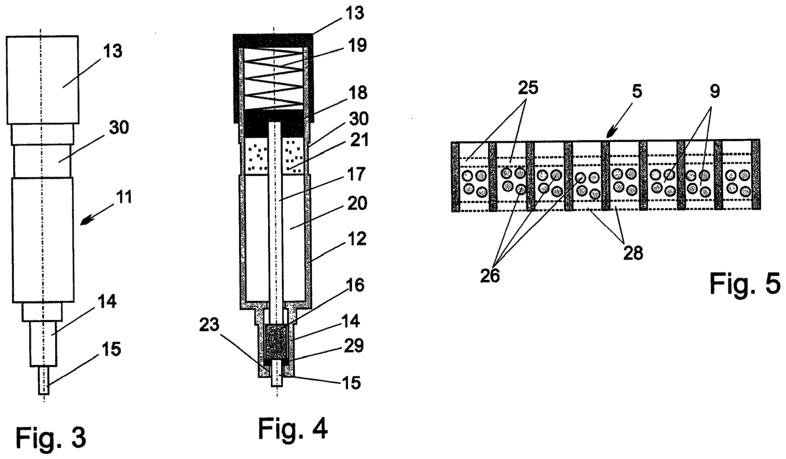

Der Synthesestift (Fig.3 und Fig.4) besitzt einen hohlzylinderförmigen Grundkörper (Reagenzienreservoir), der durch einen Schraubverschluss verschließbar ist und ein fußseitiges Mundstück, das an die freie Öffnung der Reaktionskammern in der Syntheseplatte angepasst und mit einer Auslauföffnung ausgestattet ist. Die Auslauföffnung wird durch eine Ventilnadel mit einem Sperrventil verschlossen, die durch eine Kolbenstange und einen Kolben im Grundkörper geführt und durch eine auf den Kolben wirkende Druckfeder in ihrer Schließstellung lösbar fixiert sind. Der Zylinderraum unterhalb des Kolbens dient zur Aufnahme eines einzigen Synthesebausteines und eines Schutzgases, wobei die dosierte Abgabe des Reagenzes durch einfaches Aufsetzen des Mundstückes auf dem permeablen Material, mit dem die Öffnungsseite der Reaktionskammer abgedeckt ist, erfolgt. Hierbei wird durch Eindrücken der Ventilnadel gleichzeitig das Sperrventil vom Ventilsitz gelöst und die Auslauföffnung freigegeben. Die Menge des abgegebenen Reagenzes wird von der Zeitdauer bestimmt, in der das Mundstück auf dem permeablen Material aufgesetzt ist. Mit dem Abheben des Mundstückes wird die Auslauföffnung selbsttätig wieder verschlossen.

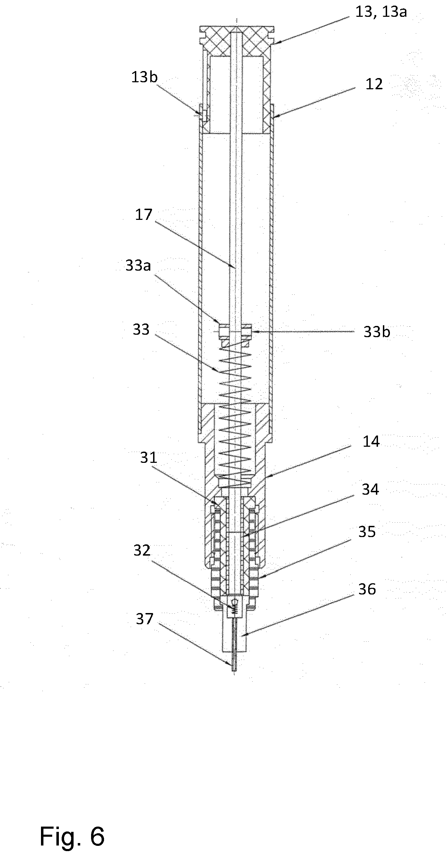

In einer weiteren Ausführung des Synthesestiftes (Fig. 6) ist der Schraubverschluss des Reagenzienreservoirs durch einen beweglichen Deckel mit Bajonettverschluss ersetzt. Eine Kolbenstange, die bevorzugt zentriert im Deckel angeordnet, insbesondere eingepresst ist, führt durch den gesamten Synthesestift in einen Dosierzylinder. Der Dosierzylinder ist, beispielsweise mit einem Rückschlagventil nach unten verschlossen. Durch Drücken des Deckels wird vermittels Kolben eine definierte Reagenzienmenge abgegeben. Ein im Synthesestift installiertes Rückstellmittel, beispielsweise eine Feder, holt den Kolben zurück. Gleichzeitig oder nachgelagert wird der Dosierzylinder wieder befüllt. Ein geeignetes Stellmittel im fußseitigen Mundstück, beispielsweise ein Rückschlagventil, stellt sicher, dass nur durch eine aktive Abgabe Lösung dosiert werden kann. Diese Ausführung des Synthesestiftes erlaubt die berührungsfreie Abgabe in den Reaktionsraum. Die geschlossene Bauweise des Synthesestiftes mit einem geschlossenen Reagenzienreservoir gewährleistet eine hohe Reagenzienstabilität.

Mit Vorteil ist das Ultraschallbad der erfindungsgemäßen Vorrichtung absenkbar beziehungsweise hebbar ausgeführt. Damit wird erreicht, dass das Reaktionsgefäß, vorzugsweise in mehreren Stufen oder aber stufenlos bis zur vorbestimmten Höhe im Ultraschallbad versenkt werden kann.

Zudem kann das Ultraschallbad für unterschiedliche Versuchsaufbauten, insbesondere unterschiedliche Synthesebehälter Anwendung finden.

Das Ultraschallbad ist ferner mit Vorteil temperierbar, insbesondere zur geregelten Erzielung eines Temperaturbereichs von 20 bis 100 °C, vorzugsweise eines Bereichs von 20 bis 70 °C, insbesondere eines Bereichs von 40 bis 60 °C ausgestaltet. Insbesondere weist die Vorrichtung eine Kühlung zur Temperaturreduzierung des Bades, beispielsweise infolge Erhitzung durch hohe Ultraschallfrequenzen auf. Dies ist insbesondere bei Verwendung von Frequenzen ab 500 kHz, insbesondere ab 1000 kHz von Vorteil.

Ferner ist das Ultraschallbad beziehungsweise dessen Ultraschallgenerator der erfindungsgemäßen Vorrichtung eingerichtet, variable Frequenzen im Hochfrequenzbereich (100 bis 2000 kHz, vorzugsweise 100 bis 500 kHz) zu generieren und auf das flüssige Bad zu übertragen. Hierzu bringt es sowohl Vorteile, wenn die unterschiedlichen Frequenzen alternativ oder aber additiv einander zuschaltbar sind.

In bevorzugter Ausgestaltung ist das Ultraschallbad, beziehungsweise jeweils der Ultraschallgenerator des Ultraschallbads mit notwendigen Leistungen im Bereich von 100 bis 300 W, vorzugsweise im Bereich von 170 bis 280 W für großtechnische Anwendungen im Bereich von 250 bis 700 W, insbesondere im Bereich von 500 bis 600 W erreicht werden.

Durch den Einsatz separater Synthesestifte für jeden Synthesebaustein und die öffnungsseitige Abdeckung der Reaktionskammer in den Syntheseplatten werden das Kontaminationsrisiko wesentlich gesenkt und Kreuzkontaminationen praktisch ausgeschlossen. Eine Verschleppung von Synthesebausteinen, wie sie häufig bei nicht ausreichenden Spülvorgängen auftraten, ist nicht mehr möglich.

Mit dem Fortfall der Spülvorgänge wird nicht nur der Verbrauch an organischen Lösungsmitteln erheblich gesenkt, sondern auch eine vielfach beschleunigte Synthese erreicht.

Die beschriebenen Ausgestaltungen sind, sofern im Einzelfall nicht anders beschrieben, vorteilhafterweise miteinander kombinierbar. Die Ausgestaltungen der Erfindung betreffen ansonsten das Verfahren und die Vorrichtung gleichermaßen.

Nachstehend soll die Erfindung an lediglich der Veranschaulichung dienenden Ausführungsbeispielen und Ergebnissen näher erläutert werden. In den dazugehörigen Zeichnungen zeigen:
- Figur 1: eine schematische Darstellung der erfindungsgemäßen Vorrichtung für die Synthese von Peptiden,
- Figur 2: die Draufsicht auf die Arbeitsfläche der Vorrichtung nach Figur 1,
- Figur 3: eine schematische Darstellung des Synthesestiftes für die separate Zuführung, Dosierung und Reagenzienbevorratung in einer bevorzugten Ausgestaltung der Erfindung,
- Figur 4: einen Längsschnitt durch den Synthesestift nach Figur 3,
- Figur 5: den Schnitt A-A aus Figur 2 durch eine Syntheseplatte mit den nach der Erfindung ausgebildeten Reaktionskammer,
- Figur 6: eine schematische Darstellung eines Längsschnitts eines Synthesestifts in einer weiter bevorzugten Ausgestaltung der Erfindung,
- Figur 7: schematische Darstellung des Ablaufs eines Verfahrens zur Festphasenpeptidsynthese gemäß einer bevorzugten Ausgestaltung der Erfindung,
- Figur 8: graphische Darstellung der Stabilität von Tryptophan mit dem erfindungsgemäßem Verfahren anhand der Synthese von Endomorphin,
- Figur 9: graphische Darstellung der Stabilität eines Acyl-Träger Proteins (ACP) mit dem erfindungsgemäßem Verfahren,
- Figur 10: graphische Darstellung der Stabilität eines Acyl-Träger Proteins (ACP) mit dem einem Vergleichsverfahren nach dem Stand der Technik,
- Figur 11: graphische Darstellung des Vergleichs der durchschnittlichen Synthesequalität des ACP-Peptids unter Berücksichtigung der Synthesestrategie,
- Figur 12: graphische Darstellung der Qualität der Synthesen gemäß Figur 11,
- Figur 13: graphische Darstellung der Überprüfung von Stocklösungen an Aminosäuren unterschiedlicher Lösungsdauer,
- Figur 14: graphische Darstellung des Vergleichs von Einfach- und Doppelkopplung,
- Figur 15: graphische Darstellung des Vergleichs von Einfach- und Doppelkopplung sowie Aminosäureüberschuss, und
- Figur 16: graphische Darstellung des Vergleichs der durchschnittlichen Synthesequalität eines Peptids unter Verwendung verschiedener Ultraschallfrequenzen.

Die in Figur 1 schematisch dargestellte Synthesevorrichtung 1 ist auf der Basis eines Laborpipettierroboters aufgebaut und besitzt einen in der x-, y- und z-Achse bewegbaren Greifarm 2. Auf der Arbeitsfläche 3 befinden sich ein Synthesegefäß, insbesondere Syntheseplatten 5, die bezüglich des Rasters und der Anordnung der Reaktionskammern 9 von an sich bekannten Mikrotiterplatten abgeleitet sind und ein 6er, 12er, 24er, 48er, 96er, 384er, 1536er oder 3456 Raster der Reaktionskammern 9 besitzen, wodurch eine hohe Parallelisierung der Synthese erreicht wird. Die Syntheseplatten 5 sind auf einen Ventilblock 6 aufgesetzt und besitzen bodenseitig eine Membran 28 aus einem porösen Material, um die verbrauchten Reagenzien und Spülflüssigkeiten aus den Reaktionskammern 9 mit Hilfe des Ventilblockes 6, der an eine Absaugpumpe angeschlossen ist, in einen Abfall abzusaugen.

Die Syntheseplatte 5 ist nebst Ventilblock 6 und Probenplatte 27 in einem, insbesondere höhenverstellbaren und steuerbar zu- und abschaltbaren Ultraschallbad 50 angeordnet. Das Ultraschallbad 50 weist ein Gefäß mit einem flüssigen Übertragungsmedium auf, in welchem das Synthesegefäß 5 angeordnet ist. Je nach Position des Ultraschallbads 50 ist ein Meniskus des Synthesegefäßes 5 eines Reaktionsmediums mindestens bis zur Hälfte, vorzugsweise mindestens bis zu Dreiviertel, insbesondere vollständig unterhalb einer Füllhöhe des Übertragungsmediums des Ultraschallbads 5. Das Ultraschallbad 50 ist eingerichtet, Ultraschall mit Frequenzen im Bereich von mindestens 25 kHz bis 2 MHz über das Übertragungsmedium zu übertragen.

Die Synthesevorrichtung 1 ist ferner mit einem oder mehreren Spülkämmen 8 ausgestattet, die über den Spülmittelzuführungen 10 mit dem entsprechenden Spülmittelreservoir verbunden sind. Um die in den Reaktionskammern 9 befindlichen Proben, an denen ein Synthesebaustein angekuppelt worden ist, nach Ablauf der Reaktionszeit und Absaugen der verbrauchten Reaktionslösung zu spülen, wird der Spülkamm 8 mit dem benötigten Spülmittel vom Greifarm 2 aufgenommen und zur dosierten Abgabe der Spülflüssigkeit über die Reaktionskammern 9 der Syntheseplatten 5 verfahren. Nach dem Spülen wird mit einem weiteren Spülkamm 8 die zur Abspaltung der temporären Schutzgruppe des angekoppelten Synthesebausteins notwendige Lösung, wie vorstehend beschrieben, zugeführt. Nach Ablauf einer Inkubationszeit wird die Abspaltlösung über den Ventilblock 6 mit Hilfe der Absaugpumpe 7 abgezogen und die Probe gewaschen. Nach dem Waschen startet ein neuer Synthesezyklus, mit dem ein weiterer Synthesebaustein angekuppelt wird.

Nach der vorliegenden Erfindung sind für jeden Synthesebaustein separate Synthesestifte 11 vorgesehen, in denen die Reagenzien 20 in einem geschlossenen Raum vorgelegt werden und mit einem Schutzgas 21 überschichtet werden können. Die einzelnen Synthesestifte 11 mit dem jeweiligen Synthesebaustein werden in einer Halterung 4 der Synthesevorrichtung 1 bereitgestellt und mit dem Greifarm 2, der die Synthesestifte 11 an der Greifarmaufnahme 30 erfasst, zur dosierten Abgabe der Reagenzien zu den Reaktionskammer 9 der Syntheseplatten 5 gebracht.

Der erfindungsgemäß verwendete Synthesestift 11 besteht aus einem hohlzylinderförmigen Grundkörper 12 mit einem fußseitigen Mundstück 14 und einem Schraubverschluss 13, der den Zylinderraum dicht verschließt. Im Mundstück 14 befindet sich eine Auslauföffnung, die durch eine Ventilnadel 15 und ein Sperrventil 16, das in Schließposition auf einer Dichtung 29 aufliegt, verschlossen ist. Ventilnadel 15 und Sperrventil 16 werden über eine Kolbenstange 17 durch einen Kolben 18 geführt. Der erforderliche Schließdruck für das Sperrventil 16 wird durch eine Druckfeder 19 erzeugt, die auf dem Kolben 18 aufliegt und sich gegen die innere Stirnfläche des Schraubverschlusses 13 abstützt. Der freie Raum unterhalb des Kolbens 18 dient für die Vorlage des jeweiligen Synthesebausteines 20, der vorteilhafterweise mit einem Schutzgas 21 überschichtet wird. So können hochreaktive Reagenzien unter einer Schutzgasatmosphäre über lange Zeiträume stabil gehalten werden, was die Qualität der Syntheseprodukte deutlich verbessert.

Um Kreuzkontaminationen bei einem direkten Kontakt des Mundstückes 14 mit der Probe sicher auszuschließen, sind erfindungsgemäß die Reaktionskammern 9, in denen sich die Proben resp. die Festphase 26, beispielweise ein Syntheseharz befinden, öffnungsseitig mit einem permeablen Material 25, beispielsweise einer Fritte, abgedeckt. Zum Ankoppeln eines Synthesebausteines 20 an die Probe resp. an das Syntheseharz wird das Mundstück 14 des Synthesestiftes 11 auf das die Reaktionskammer abschließende permeable Material 25 aufgesetzt, wodurch sich die Ventilnadel 15 entgegen dem Schließdruck der Druckfeder 19 nach innen verschiebt und das Sperrventil 16 freigegeben wird. Hiernach kann die Reagenzienlösung frei ausfließen, wobei die Dosierung der ausfließenden Lösung von der Zeitdauer bestimmt wird, in der das Mundstück 14 auf das Material 25 aufgesetzt ist.

Mit der Abspaltung der letzten temporären Schutzgruppe und Waschen der Proben erfolgt die Abspaltung der an die Festphase 26 angekoppelten Synthesebausteine 20. Hierzu wird mittels Spülkamm 8 eine Abspaltlösung zu den Proben gegeben und eine Abspaltreaktion eingeleitet. Nach Ablauf der Inkubationszeit wird der Ventilblock 6 so geschaltet, dass die in der Abspaltlösung gelösten Verbindungen in die Aufnahmekammern einer Probenplatte 27 geleitet werden, die gemäß einem weiteren Merkmal der Erfindung unter dem Ventilblock 6 angeordnet und mit einer Absauganlage verbunden ist. Die Probenplatten 27 entsprechen hinsichtlich ihres konstruktiven Aufbaus und ihrer Ausbildung den Syntheseplatten 5. Mit der Überführung der von der Festphase 26 gelösten Verbindungen in die Probenplatte 27 ist die Synthese abgeschlossen.

Figur 6 zeigt eine weiter bevorzugte Ausgestaltung des Synthesestifts 11, wobei gleiche Bezugszeichen einander entsprechen. Der Schraubverschluss des Reagenzienreservoirs ist in dieser Ausgestaltung durch einen beweglichen Deckel 13a mit Bajonettverschluss ersetzt. Eine Kolbenstange 17, die bevorzugt zentriert im Deckel angeordnet, insbesondere eingepresst ist führt durch den gesamten Synthesestift in einen Dosierzylinder 31. Der Dosierzylinder 31 ist, beispielsweise mit einem Rückschlagventil 32 nach unten verschlossen. Durch drücken des Deckels 13a wird vermittels Kolben ein definierte Reagenzienmenge abgegeben. Ein im Synthesestift 11 installiertes Rückstellmittel, beispielsweise eine Feder 33, 33a, 33b holt den Kolben zurück. Gleichzeitig oder nachgelagert wird der Dosierzylinder wieder befüllt 34. Ein geeignetes Stellmittel im fußseitigen Mundstück 14, beispielsweise ein Rückschlagventil stellt sicher, dass nur durch eine aktive Abgabe Lösung dosiert werden kann. Diese Ausführung des Synthesestiftes 11 erlaubt die berührungsfreie Abgabe in den Reaktionsraum. Die geschlossene Bauweise des Synthesestiftes 11 mit einem geschlossenen Reagenzienreservoir gewährleistet eine hohe Reagenzienstabilität.

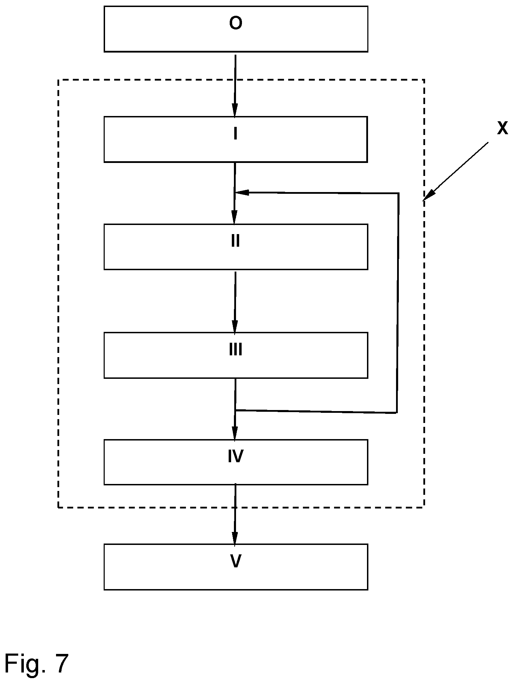

Figur 7 zeigt eine schematische Darstellung des erfindungsgemäßen Verfahrens.

Das erfindungsgemäße Verfahren ist Teil einer Festphasenpeptidsynthese, wie sie beispielsweise durch die erfindungsgemäße Vorrichtung durchgeführt wird beziehungsweise durchführbar ist. Hierzu wird der N-Terminus einer Aminosäure mit einer Schutzgruppe vor unerwünschten Reaktionen geschützt. Die so geschützte Aminosäure wird über ihren C-Terminus an ein festes Trägermaterial gebunden (I). Anschließend wird der N-Terminus entschützt (II) um mittels Peptidpropagation eine weitere am N-Terminus geschützte Aminosäure an den N-Terminus der vorherigen Aminosäure zu binden (III). Die Schritte II bis III werden so oft wiederholt, bis die gewünschte Kettenlänge an Aminosäuren erreicht ist. Mit erreichter Kettenlänge wird die Reaktion in einem Schritt IV durch Abspalten des Peptids vom Trägermaterial abgebrochen. Zumindest abschließend wird das Peptid mit einem geeigneten Lösungsmittel gewaschen (Schritt V). Optional erfolgt zu Beginn des Verfahrens ein Vorquellen (Schritt O) des festen Trägermaterials, meist ein Harz. Erfindungsgemäß ist zumindest einer der genannten Schritte zumindest zeitweise ultraschallunterstützt (X). Darunter ist zu verstehen, dass zumindest zeitweise Ultraschall (X) einer Frequenz von mindestens mehr als 100 kHz auf das Reaktionsmedium, in dem die Synthese stattfindet, einwirkt.

Es zeigte sich, dass ein Ultraschallbad, in dem das Reaktionsmedium mittels eines Gefäßes eingebracht ist, besonders gut zur Übertragung geeignet ist. Sowohl präparativ, als auch in Bezug auf die Synthesezeit zeigte es sich ferner vorteilhaft, wenn Ultraschall (X) über mehrere Schritte hinweg, vorzugsweise ohne Abschaltung zwischen den Schritten auf das Reaktionsmedium einwirkt. Insbesondere bezogen auf die Schritte I bis IV kann der erfindungsgemäß durchgeführte Ultraschall eine Reduktion der Synthesezeit im Bereich einer Größenordnung bewirken.

In Tabelle 1 sind die Synthesezeiten der einzelnen Schritte der Wiederholeinheiten für ein Verfahren nach dem Stand der Technik ohne Ultraschalleinwirkung und gemäß der Erfindung mit Ultraschalleinwirkung im Bereich von 50 kHz (Referenzbereich) bis 150 kHz gegenübergestellt. Es wird deutlich, dass das erfindungsgemäße Verfahren um ein Zehnfaches schneller ist als ein vergleichbares Verfahren ohne Ultraschall.

**Tabelle 1: Vergleich der benötigten Synthesezeiten von Verfahren nach Stand der Technik und gemäß der Erfindung.**

| Schritt | Stand der Technik | Erfindungsgemäßes Verfahren |
|---|---|---|
| Entschützen (Schritt II) | 3 x 5min | 2 x 1min |
| Waschen (IV) | 5 x 1min | 3 x 30sec |
| Peptidpropagation (III) | 2 x 30min | 2 x 3min |
| Summe | 1h 20min | 8min 30sec |

Die Aminosäuren können neben den am N- bzw- C-Terminus gebundenen Schutzgruppen weitere Schutzgruppen zur Blockierung reaktiver Seitenketten aufweisen. Dabei ist auf die Anforderungen bezüglich der chemischen und physikalischen Umgebung bei der Peptidsynthese, wie Ultraschall und Basen- bzw. Säurestabilität zu achten. Geeignete Schutzgruppen für reaktive Seitenketten zur Verwendung im erfindungsgemäßen Verfahren sind beispielsweise säurelabile Schutzgruppen, beispielsweise S-2,4,6-Trimethoxybenzyl (Tmob), Triphenylmetyl (Trt), tert-Butyl (tBu), tert-Butyloxycarbonyl (Boc) und 2,2,4,6,7-Pentamethyldihydrobenzofuran-5-sulfonyl (Pbf).

Bezogen auf das erfindungsgemäße Verfahren sind beispielsweise Fmoc-Aminosäuren ausgewählt aus der folgenden die jeweilige Aminosäure als Single-letter-code notierten Gruppe zur Peptidpropagation besonders geeignet: Fmoc-A-OH, Fmoc-C(Trt)-OH, Fmoc-D(OtBu)-OH, Fmoc-E(OtBu)-OH, Fmoc-F-OH, Fmoc-G-OH, Fmoc-H(Trt)-OH, Fmoc-I-OH, Fmoc-K(Boc)-OH, Fmoc-L-OH, Fmoc-M-OH, F moc-N(Trt)-OH, Fmoc-P-OH, Fmoc-Q-Trt-OH, Fmoc-R-Pbf-OH, Fmoc-S-tBu-OH, Fmoc-T-tBu-OH, Fmoc-V-OH, Fmoc-W(Boc)-OH, Fmoc-Y-(tBu)-OH, Fmoc-Gln(Tmob)-OH, Fmoc-Asn(Tmob)-OH (TMOB = 2,4,6-Trimethoxybenzyl).

Die Fmoc- Aminosäuren können sowohl in der L- als auch in der D-Form vorliegen.

Bei diesen konnte die Synthesezeit ohne Ausbeuteverluste um mehr als das Zehnfache im Vergleich zum Stand der Technik reduziert werden.

Tabelle 2 zeigt den typischen Syntheseverlauf des erfindungsgemäßen Verfahrens in einer bevorzugten Ausgestaltung anhand eines Pipettierschemas am Beispiel von Endomorphin. Dies umfasst im Wesentlichen die oben genannten Schritte (O-V), die im folgenden Beispiel jedoch detailiierter und mit Unterschritten beschrieben sind. Ein erster Schritt ist das Vorquellen des Harzes (O), gefolgt vom Entschützen des Harzes beispielsweise mit 20 % Piperidin in DMF, anschließendem Waschen mit einem Lösungsmittel, beispielsweise DMF oder DCM, Kopplung der Aminosäure (I), erneutem Waschen mit einem Lösungsmittel, beispielsweise DMF oder DCM, Entschützen der Aminosäure und abschließend Waschen mit einem Lösungsmittel, vorzugsweise DMF oder DCM.

Der Zyklenablauf ist dabei stets gleich. Nach dem Koppeln der letzten Aminosäure (AA) wird entschützt, gewaschen und mit Lösungsmittel, beispielsweise DMF oder DCM gespült. Während der einzelnen Zyklen wirkt in der gezeigten Ausführung Ultraschall mit Frequenzen im Bereich von 25 kHz bis 2 MHz auf das Reaktionsmedium ein. Im vorliegenden Beispiel wird hierzu der Ultraschall (X) auch zwischen den Schritten nicht unterbrochen. Alternativ kann der Ultraschall zwischen den Schritten oder während einzelner Schritte ausgesetzt werden. In den getesteten Frequenzen im Bereich von 40 kHz bis 2 MHz und insbesondere im Bereich von 50 kHz bis 200 kHz zeigte sich jedoch die kontinuierliche Beschallung als besonders vorteilhaft.

Die Schritte Vorquellen und abschließendes Spülen mit Lösungsmittel, vorzugsweise Dichlormethan (DCM) erfolgen in dem gezeigten Beispiel ohne Ultraschalleinwirkung. Dies ist jedoch lediglich eine bevorzugte Ausführung, so dass auch die Beschallung über alle Schritte erfolgen kann.

**Tabelle 2: Syntheseverlauf einer Festphasenpeptidsynthese mit dem erfindungsgemäßen Verfahren in einer bevorzugten Ausgestaltung.**

| | | Pipettierschema | | | |
|---|---|---|---|---|---|
| | | Volumen (ml) | t (min) | Wiederholung | Ultraschall |
| | | **1. AA, Phe, F** | | | |
| 1 | Vorquellen | 2 | 2 | 2 | nein |
| 2 | Entschützen | 1 | 1 | 2 | ja |
| 3 | Waschen | 1 | 30s | 3 | ja |
| 4 | Koppeln | F in HCTU, 250 µl | 3 | 2 | ja |
| | | DIPEA, 153,3 µl | | | |
| 5 | Waschen | 1 | 30s | 3 | ja |

| | | **2. AA, Trp, W** | | | |
|---|---|---|---|---|---|
| 6 | Entschützen | 1 | 1 | 2 | ja |
| 7 | Waschen | 1 | 30s | 3 | ja |
| 8 | Koppeln | W in HCTU, 250 µl | 3 | 2 | ja |
| | | DIPEA, 153,3 µl | | | |
| 9 | Waschen | 1 | 30s | 3 | ja |

| | | **3. AA, Pro, P** | | | |
|---|---|---|---|---|---|
| 10 | Entschützen | 1 | 1 | 2 | ja |
| 11 | Waschen | 1 | 30s | 3 | ja |
| 12 | Koppeln | P in HCTU, 250 µl | 3 | 2 | ja |
| | | DIPEA, 153,3 µl | | | |
| 13 | Waschen | 1 | 30s | 3 | ja |

| | | **4. AA, Tyr, Y** | | | |
|---|---|---|---|---|---|
| 14 | Entschützen | 1 | 1 | 2 | ja |
| 15 | Waschen | 1 | 30s | 3 | ja |
| 16 | Koppeln | Y in HCTU, 250 µl | 3 | 2 | ja |
| | | DIPEA, 153,3 µl | | | |
| 17 | Waschen | 1 | 30s | 3 | ja |
| 18 | Entschützen | 1 | 1 | 2 | ja |
| 19 | Waschen | 1 | 30s | 3 | ja |
| 36 | Spülen mit DCM | 1 | 1 | 3 | nein |

Das erfindungsgemäße Verfahren ist als sogenannte Kurzzeit- oder als Langzeitsynthese durchführbar. Der Unterschied zwischen den beiden ist exemplarisch in Tabelle 3 dargestellt:

**Tabelle 3: Beschreibung Kurz- und Langzeitsynthese**

| **Kurzzeitsynthese** | **Langzeitsynthese** |
|---|---|
| Entschützen mit Ultraschall | Entschützen mit Ultraschall |
| Entschützen Dauer: 30 s | Entschützen Dauer: 1 min |
| Anzahl an Entschützschritten: 1 | Anzahl an Entschützschritten: 2 |
| Waschen ohne Ultraschall | Waschen mit Ultraschall je 30 s |
| Pro Waschschritt: 5x waschen | Pro Waschschritt: 3x waschen |
| Einfachkopplung der AA | Doppelkopplung der AA |

Die Kurzzeitsynthese unterscheidet sich im Wesentlichen darin von der Langzeitsynthese, dass die Dauer beim Entschützen halbiert ist. Ferner ist die Anzahl der Wasch- und Entschützungsschritte reduziert. Trotzdem die Langzeitsynthese eine längere Synthesezeit beansprucht, liefert auch diese eine im Vergleich zum Stand der Technik auf ein Zehntel reduzierte Synthesezeit.

### Syntheseschema für die Doppelkopplung bei der Ultraschallsynthese (Einzelfrequenz)

**Tabelle 4**

| **Schritt** | **Chemikalie** | **Fmoc-AA** | **V [ml]** | **t [min]** |
|---|---|---|---|---|
| entschützen | 20% Piperidin | | 1 | 1 |
| absaugen | | | | |
| entschützen | 20% Piperidin | | 1 | 1 |
| absaugen | | | | |
| waschen | DMF | | 1 | 0,5 |
| absaugen | | | | |
| waschen | DMF | | 1 | 0,5 |
| absaugen | | | | |
| waschen | DMF | | 1 | 0,5 |
| absaugen | | | | |
| Zugabe AA (+ HCTU, DIPEA) | AA + 1ml HCTU + 1ml DIPEA | G | 2 | 3 |
| absaugen | | | | |
| Zugabe AA (+ HCTU, DIPEA) | AA + 1ml HCTU + 1ml DIPEA | G | 2 | 3 |
| absaugen | | | | |
| waschen | DMF | | 1 | 0,5 |
| absaugen | | | | |
| waschen | DMF | | 1 | 0,5 |
| absaugen | | | | |
| waschen | DMF | | 1 | 0,5 |
| absaugen | | | | |

| | | | | |
|---|---|---|---|---|
| Gesamtdauer eines Zyklus bei der Doppelkopplung: 11min | | | | |

Dieser Zyklus wird solange wiederholt, bis gesamte Sequenz synthetisiert wurde (Beispiel ACP: H-VQAAIDYING-NH2 --> 10 Aminosäuren → 10 Zyklen).

Die Vorbereitenden Schritte wie Vorquellen und waschen, sowie die abschließenden Waschschritte sind hier nicht aufgeführt

### Syntheseschema für die Einfachkopplung bei der Ultraschallsynthese (Einzelfrequenz)

**Tabelle 5**

| **Schritt** | **Chemikalie** | **Fmoc-AA** | **V [ml]** | **t [min]** |
|---|---|---|---|---|
| entschützen | 20% Piperidin | | 1 | 1 |
| absaugen | | | | |
| entschützen | 20% Piperidin | | 1 | 1 |
| absaugen | | | | |
| waschen | DMF | | 1 | 0,5 |
| absaugen | | | | |
| waschen | DMF | | 1 | 0,5 |
| absaugen | | | | |
| waschen | DMF | | 1 | 0,5 |
| absaugen | | | | |
| Zugabe AA (+ HCTU, DIPEA) | AA + 1ml HCTU + 1ml DIPEA | G | 2 | 3 |
| absaugen | | | | |
| waschen | DMF | | 1 | 0,5 |
| absaugen | | | | |
| waschen | DMF | | 1 | 0,5 |
| absaugen | | | | |
| waschen | DMF | | 1 | 0,5 |
| absaugen | | | | |

| | | | | |
|---|---|---|---|---|
| Gesamtdauer eines Zyklus bei der Einfachkopplung: 8min | | | | |

Dieser Zyklus wird solange wiederholt, bis gesamte Sequenz synthetisiert wurde (Beispiel ACP: H-VQAAIDYING-NH2 → 10 Aminosäuren → 10 Zyklen).

Die Vorbereitenden Schritte wie Vorquellen und waschen, sowie die abschließenden Waschschritte sind hier nicht aufgeführt

### Syntheseschema für die Einfachkopplung bei der Ultraschallsynthese (mehrere Frequenzen Bsp: 132kHz und 470kHz)

**Tabelle 6**

| **Schritt** | **Chemikalie** | **Fmoc-AA** | **Volumen [ml]** | **Zeit [min]** | **Ultraschall** | **F [kHz]** |
|---|---|---|---|---|---|---|
| entschützen | 20% Piperidin | | 1 | 2*0,5 | ja | 132 + 470 |
| Absaugen | | | | | | |
| entschützen | 20% Piperidin | | 1 | 2*0,5 | ja | 132 + 470 |
| Absaugen | | | | | | |
| Waschen | DMF | | 1 | 0,5 | ja | 132 |
| Absaugen | | | | | | |
| Waschen | DMF | | 1 | 0,5 | ja | 132 |
| Absaugen | | | | | | |
| Waschen | DMF | | 1 | 0,5 | ja | 132 |
| Absaugen | | | | | | |
| Zugabe AA (+ HCTU, DIPEA) | AA + 1ml HCTU + 1ml DIPEA | I | 2 | 1+2 | ja | 132 + 470 |
| Absaugen | | | | | | |
| Waschen | DMF | | 1 | 0,5 | ja | 132 |
| Absaugen | | | | | | |
| Waschen | DMF | | 1 | 0,5 | ja | 132 |
| Absaugen | | | | | | |
| Waschen | DMF | | 1 | 0,5 | ja | 132 |
| Absaugen | | | | | | |

| | | | | | | |
|---|---|---|---|---|---|---|
| Entschützen: 2*0,5 ja 132 + 470 --> 0,5min bei 132kHz und anschließend 0,5min bei 470kHz Zugabe AA (+HCTU, DIPEA): 1+2 ja 132 + 470 → 1min bei 132kHz und anschließend 2min bei 470kHz Gesamtdauer eines Zyklus bei der Einfachkopplung: 8min | | | | | | |

Dieser Zyklus wird solange wiederholt, bis gesamte Sequenz synthetisiert wurde (Beispiel: H-PYLFWLAAI-NH2 → 9 Aminosäuren → 9 Zyklen).

Die vorbereitenden Schritte wie Vorquellen und waschen, sowie die abschließenden Waschschritte sind hier nicht aufgeführt.

### Syntheseschema für die LIPS-Synthese (3fach-Kopplung)

**Tabelle 7**

| **Schritt** | **Chemikalie** | **Zeit** | **Anzahl** | **Gesamtzeit** |
|---|---|---|---|---|
| entschützen | 20% Piperidin | 2min | 5 | 10min |
| absaugen | | | | |
| waschen | DMF | 10sec | 5 | 50sec |
| absaugen | | | | |
| Zugabe AA | Verteilung der Stifte | ca 20min | 3 | 105min |
| Aktivierung | NMM | 15min | | |
| absaugen | | 10sec | | |
| waschen | DMF | 10sec | 3 | 30sec |
| absaugen | | | | |
| Acetylierung | Capping Lösung | 2min | 2 | 4min |
| absaugen | | | | |
| waschen | DMF | 20sec | 5 | 2min |
| absaugen | | | | |

| | | | | |
|---|---|---|---|---|
| Gesamtdauer eines Zyklus bei der 3fach-Kopplung: ca 122min | | | | |

Dieser Zyklus wird solange wiederholt, bis gesamte Sequenz synthetisiert wurde (Beispiel: H-VQAAIDYING-NH2 --) 10 Aminosäuren -7 10 Zyklen).

Die Dauer für das Verteilen der Stifte ist von mehreren Faktoren abhängig, daher hier nur eine ca.-Angabe.

### Syntheseschema für die ABI-Synthese (1fach-Kopplung) ohne Capping (Acetylierung)

**Tabelle 8**

| **Programm-Modul** | **Vorgang** | **Dauer (ca-Angabe aus dem Handbuch)** | **Bemerkung** |
|---|---|---|---|
| B | Entschützen | 15min | 2x mindestens |
| A | Lösen der Aminosäure in Kartusche | 8min | |
| D | waschen | 2,5min | Dauer variiert je nach Anzahl der durchlaufenden Zyklen |
| | | | 5x mindestens |
| E | Überführung der gelösten Aminosäure in Reaktionsgefäß | 2,1min | |
| F | Kopplung | 15min | |
| D | waschen | 2,5min | Dauer variiert je nach Anzahl der durchlaufenden Zyklen |
| | | | 5x mindestens |

| | | | |
|---|---|---|---|
| Gesamtdauer eines Zyklus bei der 1fach-Kopplung: ca 80min. | | | |

Die Zeitangaben können nur mit ca-Werten angegeben werden, da die einzelnen Module unterschiedliche Länge haben können, was widerum von der zu synthetisierenden Sequenz abhängig ist. Zudem messen interne Sensoren während des Entschützens den Anteil an entschützen Fmoc-Gruppen.

Dieser Zyklus wird solange wiederholt, bis gesamte Sequenz synthetisiert wurde ( Beispiel: H-VQAAIDYING-NH2 --> 10 Aminosäuren --> 10 Zyklen).

Innerhalb der Module sind zusätzlich Waschschritte enthalten, sodass diese nicht separat dargestellt werden.

### Syntheseschema für die ABI-Synthese (2fach-Kopplung) mit Capping (Acetylierung)

**Tabelle 9**

| **Programm-Modul** | **Vorgang** | **Dauer (ca-Angabe aus dem Handbuch)** | **Bemerkung** |
|---|---|---|---|
| B | Entschützen | 15min | 2x mindestens |
| A | Lösen der Aminosäure in Kartusche | 8min | |
| D | waschen | 2,5min | Dauer variiert je nach Anzahl der durchlaufenden Zyklen |
| | | | 5x mindestens |
| E | Überführung der gelösten Aminosäure in Reaktionsgefäß | 2,1min | |
| A | Lösen der Aminosäure in Kartusche | 8min | |
| D | waschen | 2,5min | Dauer variiert je nach Anzahl der durchlaufenden Zyklen |
| | | | 5x mindestens |
| E | Überführung der gelösten Aminosäure in Reaktionsgefäß | 2,1min | 2x |
| F | Kopplung | 15min | 2x |
| C | Capping | 9,5min | |
| D | waschen | 2,5min | Dauer variiert je nach Anzahl der durchlaufenden Zyklen |
| | | | 5x mindestens |

| | | | |
|---|---|---|---|
| Gesamtdauer eines Zyklus bei der 2fach-Kopplung: ca 130min. | | | |

Die Zeitangaben können nur mit ca-Werten angegeben werden, da die einzelnen Module unterschiedliche Länge haben können, was widerum von der zu synthetisierenden Sequenz abhängig ist. Zudem messen interne Sensoren während des Entschützens den Anteil an entschützen Fmoc-Gruppen.

Dieser Zyklus wird solange wiederholt, bis gesamte Sequenz synthetisiert wurde (Beispiel: H-VQAAIDYING-NH2 --> 10 Aminosäuren --> 10 Zyklen.

Innerhalb der Module sind zusätzlich Waschschritte enthalten, sodass diese nicht separat dargestellt werden.
ACP H-VQAAIDYING-NH2 M = 1063,2Da
Synthesemaßstab: 25µmol
Syntheseharz: Knorr Amid Harz LS 1%DVB
Aktivator: HCTU
Base: DIPEA

### Verwendete Aminosäuren:

| **Aminosäure (L-Aminosäuren)** | **Permanente Schutzgruppe** |
|---|---|
| Fmoc-Ala-OH | - |
| Fmoc-Asp(tBu)-OH | Tert. Butyl |
| Fmoc-Gly-OH | - |
| Fmoc-Ile-OH | - |
| Fmoc-Asn(Trt)-OH | Trityl |
| Fmoc-Gln(Trt)-OH | Trityl |
| Fmoc-Val-OH | - |
| Fmoc-Tyr(tBu)-OH | Tert. Butyl |

| | |
|---|---|
| Verhältnis freie Aminofunktion Harz: Aminosäure: Aktivator: Base; 1:4:3,9:8 | |

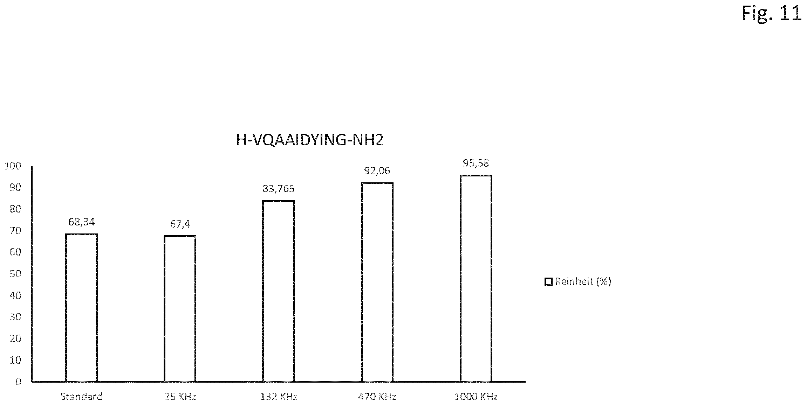

In Figur 11 wird die durchschnittliche Synthesequalität des ACP-Peptids H-VQAAIDYING-NH₂ unter Berücksichtigung der Synthesestrategie verglichen:

| | |
|---|---|
| 660-SL3 LIPS: Kopplung (Standardprotokoll) | ACP in Mikrotiterplatte (MTP) LIPS-Roboter 3fach-Dauer: 22,5h |
| USPS 132kHz 50% Leistung: aus 2 Ansätzen) | ACP im Ultraschall, 132kHz 1fach-Kopplung (Mittelwert Dauer: 2,5h |
| USPS 470kHz 50% Leistung: aus 2 Ansätzen) | ACP im Ultraschall, 470kHz 1fach-Kopplung (Mittelwert Dauer: 2,5h |
| USPS 1000kHz 60% Leistung: aus 2 Ansätzen) | ACP im Ultraschall, 1000kHz 1fach-Kopplung (Mittelwert Dauer. 2,5h |

Es ist festzustellen, dass die Höhe der Frequenz die Produktqualität vergrößert.

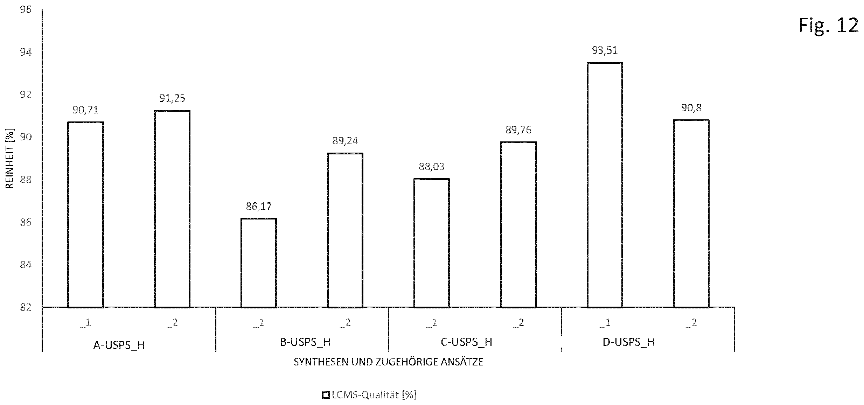

In Figur 12 sind die Qualitäten der Synthesen von H-VQAAIDYING-NH₂ 25µmol gemäß Figur 11 graphisch dargestellt.

| Batchnummer | Frequenz [kHz] | Leistung Ultraschall [%] | Ultraschall während waschen ? | Ultraschall während Entschützen? | Ultraschall während Kopplung? | Kopplungsanzahl |
|---|---|---|---|---|---|---|
| A-USPS_H_1 | 132 | 50 | ja | ja | ja | 2 |
| A-USPS_H_2 | 132 | 50 | ja | ja | ja | 2 |
| B-USPS_H_1 | 132 | 50 | nein | ja | ja | 2 |
| B-USPS_H_2 | 132 | 50 | nein | ja | ja | 2 |
| C-USPS_H_1 | 132 | 50 | nein | nein | ja | 2 |
| C-USPS_H_2 | 132 | 50 | nein | nein | ja | 2 |
| D-USPS_H_1 | 470 | 100 | ja | ja | ja | 2 |
| D-USPS_H_2 | 470 | 100 | ja | ja | ja | 2 |

Im Ergebnis ist festzustellen, dass ein dauerhaftes Schallen die Produktqualität vergrößert und dass eine Steigerung der Frequenz ebenfalls die Produktqualität erhöht.

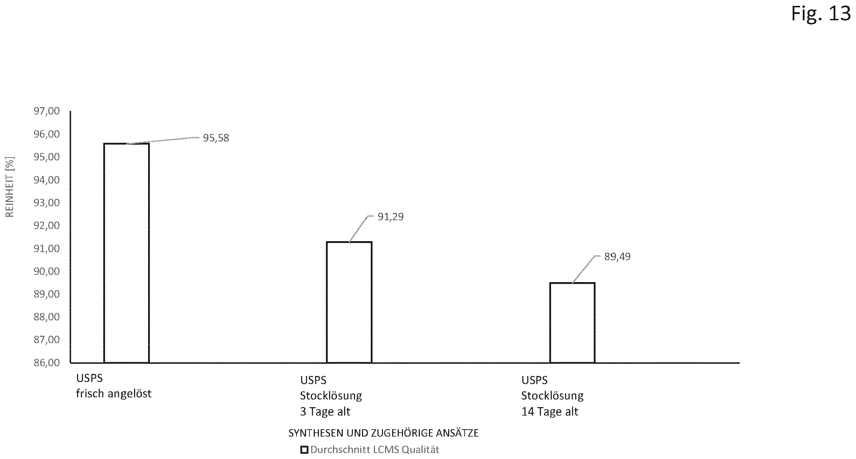

Figur 13 zeigt die Überprüfung von Stocklösungen an Aminosäuren unterschiedlicher Lösungsdauer.

Mit den unterschiedlichen Stocklösungen erfolgen Synthesen des ACP-Peptides mittels Ultraschall bei 1000kHz mit unterschiedlicher Lösungsdauer der verwendeten Aminosäuren.

Es zeigt sich, dass eine kürzere Lösungsdauer der verwendeten Aminosäuren die Produktqualität steigert.

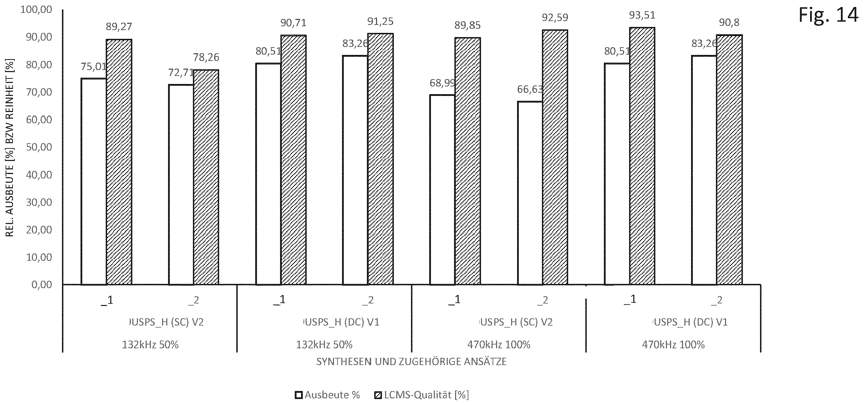

Figur 14 ist eine graphische Darstellung des Vergleichs der Ausbeute und Qualität in Bezug auf Kopplungszahl bei verschiedenen Frequenzen H-VQAAID.

| **Synthesenummer (Anzahl Ansätze)** | **Kopplungszahl** | **Frequenz Ultraschall** |
|---|---|---|
| USPS_H (2) | 1 | 132kHz 50% |
| USPS_H (2) | 2 | 132kHz 50% |
| USPS_H (2) | 1 | 470kHz 100% |
| USPS_H (2) | 2 | 470kHz 100% |

Es zeigt sich, dass bei niedrigen Frequenzen (132kHz) die LCMS Qualität mit Erhöhung der Kopplungszahl steigt.

Bei hohen Frequenzen (470kHz) erkennt man kaum einen Unterschied in der LCMS Qualität.

Frequenzunabhängig jedoch steigt die Ausbeute mit Erhöhung der Kopplungszahl.

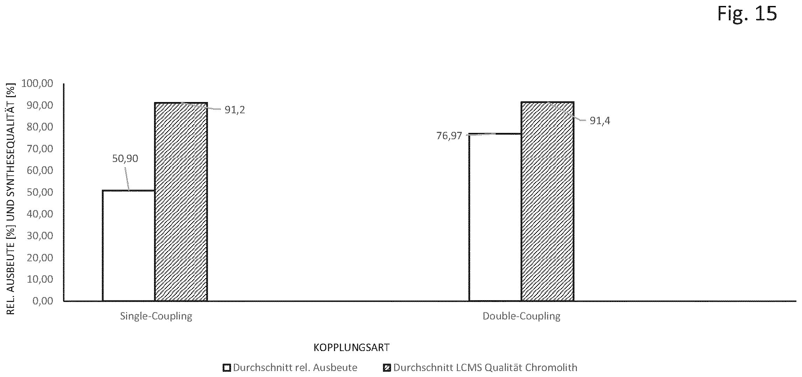

Für das Experiment (Figur 15) wird ein anderes Peptid gewählt als für die vorhergehenden Untersuchungen. Die Sequenz lautet PYLFWLAAI-NH2

Hierbei handelt es sich ebenfalls um ein schwierig zu synthetisierendes Peptid.
ACP H-PYLFWLAAI-NH2 M = 1092,6Da
Synthesemaßstab: 25µmol
Syntheseharz: Knorr Amid Harz LS 1%DVB
Aktivator: HCTU
Base: DIPEA

### Verwendete Aminosäuren:

| **Aminosäure (L-Aminosäuren)** | **Permanente Schutzgruppe** |
|---|---|
| Fmoc-Ala-OH | - |
| Fmoc-Phe-OH | - |
| Fmoc-Ile-OH | - |
| Fmoc-Leu-OH | - |
| Fmoc-Pro-OH | - |
| Fmoc-Trp(Boc)-OH | Butyloxycarbonyl |
| Fmoc-Tyr(tBu)-OH | Tert. Butyl |

| | |
|---|---|
| Verhältnis freie Aminofunktion Harz: Aminosäure: Aktivator: Base; 1:4:3,9:8 | |

In Figur 15 ist graphisch der Vergleich von Einfach- und Doppelkopplung sowie Aminosäureüberschuss für dieses Peptid dargestellt.

| **Synthesenummer (Anzahl Ansätze)** | **Kopplungszahl** | **Frequenz Ultraschall** |
|---|---|---|
| 150819USPS_H (2) | 1 (Single-Coupling) | 470kHz 50% |
| 241019USPS_H (2) | 2 (Double-Coupling) | 470kHz 50% |

Bei gleicher Frequenz ergibt sich kein signifikanter Unterschied hinsichtlich der Synthesequalität.

Bei Erhöhung der Kopplungszahl jedoch zeigt sich eine deutliche Erhöhung der relativen Ausbeute.

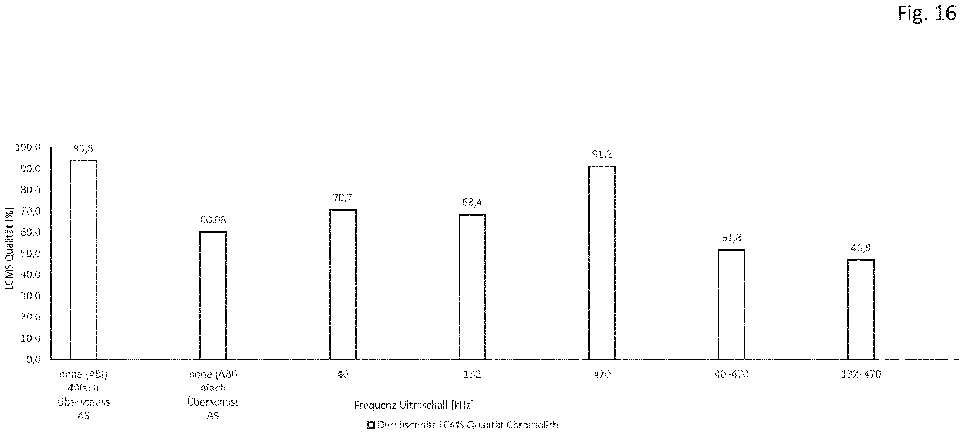

In Figur 16 wird eine graphische Darstellung des Vergleichs der durchschnittlichen Synthesequalität eines Peptids unter Verwendung verschiedener Ultraschallfrequenzen gezeigt.

Dargestellt sind die Synthesen des Peptides PYLFWLAAI-NH2, die mittels Einfachkopplung zu unterschiedlichen Ultraschallfrequenzen synthetisiert wurden.

### Ohne Ultraschall:

Konventionelle ABI Synthese mit 40fach Überschuss an Aminosäure.
Konventionelle ABI Synthese mit 4fach Überschuss an Aminosäure.

Je niedriger der Überschuss an Aminosäure, desto schlechter die LCMS-Qualität bei einer konventionellen ABI Synthese.

### Einfache Ultraschallfrequenzen 4fach Überschuss an Aminosäure

Frequenzen: 40kHz, 132kHz, 470kHz

Mit steigender Frequenz steigt die LCMS-Qualität.

### Gekoppelte Ultraschallfrequenzen (Entschützen, Kopplung), waschen ausschließlich bei niedrigerer Frequenz

Gekoppelte Frequenzen: 40kHz + 470kHz sowie 132kHz + 470kHz

Der Wechsel zwischen den Frequenzen führt zu einer signifikanten Verschlechterung der Synthesequalität.

### Ultraschall vs. konventionelle ABI Synthese

Um bei der konventionellen ABI Synthese eine gute bis sehr gute LCMS-Qualität zu erreichen, sind sehr hohe Überschüsse an Aminosäure nötig (40fach).

Mit Hilfe des Ultraschalls ist ein 4fach Überschuss an Aminosäure ausreichend. Dabei gilt, je höher die eingesetzte Frequenz, desto höher die LCMS-Qualität.

Gleichwertige Ergebnisse zeigten folgende Parameter:
ABI (40fach Überschuss) sowie 470kHz (4fach Überschuss).

Mittels der Ultraschallsynthese lassen sich in kürzerer Zeit und unter verminderten Einsatz von Lösemitteln und Aminosäuren mindestens gleichwertige, in der Regel bessere Resultate erzielen.

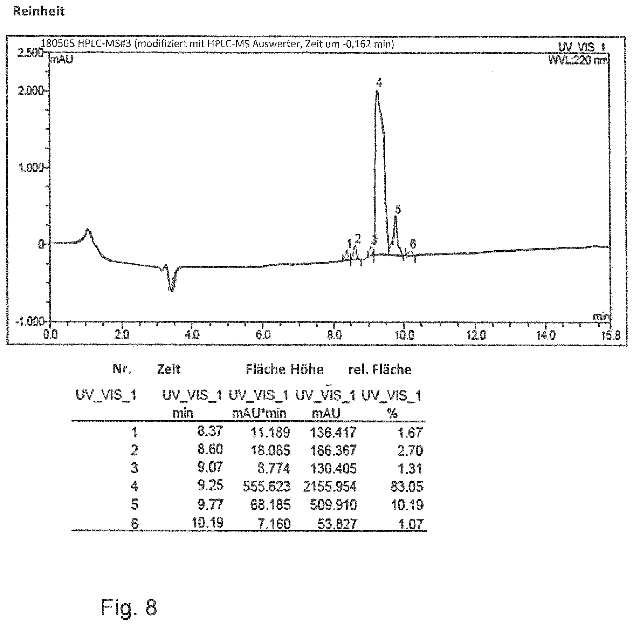

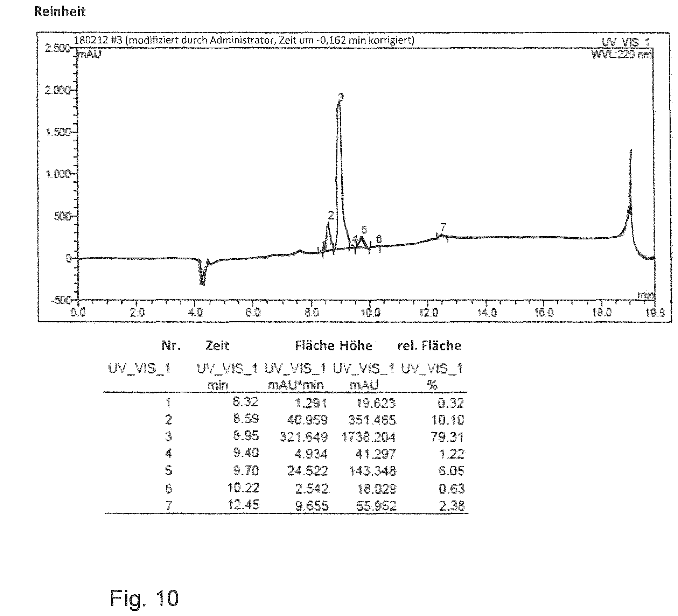

Die Figuren 8 bis 10 zeigen jeweils die Zusammensetzung eines mittels Festphasenpeptidsynthese dargestellten Peptids. Dabei wurden die in den Figuren 8 bis 9 gezeigten Peptide mittels des erfindungsgemäßen Verfahrens hergestellt, während Figur 10 ein nach dem Stand der Technik mittels Tetras synthetisiertes Peptid zugrunde liegt. Alle Verfahren wurden mit der erfindungsgemäßen Vorrichtung durchgeführt.

Die aus den jeweiligen Verfahren gewonnen Produkte wurden mittels HPLC aufgetrennt und die einzelnen Peaks mittels Massenspektrometrie und UV-Vis Spektrometrie zugeordnet. Dabei wurden Apparaturen mit den folgenden Kennzahlen verwendet:
HPLC MS System
Dionex Binäre HPLC Pumpe
Laufmittel A: Wasser plus 0,1 % Ameisensäure
Laufmittel B: Acetonitril plus 0,1 % Ameisensäure
Fluß: 0,5 ml/min
Gilson Probengeber für bis zu 4 Mikrotiterplatten
Dionex Säulenofen
Temperatur: 30°C
Dionex UV Detektor
Messung bei 220 nm
Dionex/Thermo Finnigan Surveyor MSQ Single Quadrupole Mass Spectrometer
Ionisations Modus: ESI
Probe Temperatur: 350 °C
Konus Spannung: 50 V
HPLC Trennsäule: Merk, Chromolith WP300, RP18, 100-4,6 mm

| Zeit (min) | % Laufmittel B |
|---|---|
| 0 | 5 |
| 2 | 5 |
| 12 | 100 |
| 14 | 100 |
| 15 | 5 |

Der Figur 8 ist zu entnehmen, dass die oben genannten Schutzgruppen zur Blockierung der reaktiven Seitenketten im erfindungsgemäßen Verfahren stabil sind. Hierzu sind exemplarisch für drei der gängigsten Schutzgruppen die Ergebnisse einer Peptidsynthese gemäß dem erfindungsgemäßen Verfahren gezeigt.

Figur 8 zeigt die Analyseergebnisse einer Endomorphinsynthese, die gemäß dem erfindungsgemäßen Verfahren auf Basis der Langzeitsynthesevorschrift durchgeführt wurde. Theoretische Überlegungen ließen zunächst vermuten, dass das oxidationsempfindliche Tryptophan durch den Ultraschall während der Synthese oxidieren könnte. Dies wurde jedoch nicht bestätigt. Vielmehr war die Synthese mit einer Reinheit von 83% erfolgreich. Es wurden nur wenige Nebenprodukte identifiziert.

Ebenfalls stabil während des erfindungsgemäßen Verfahrens zeigten sich in Einzelversuchen Methionin-, sowie Trityl- und Tmob-Schutzgruppen.

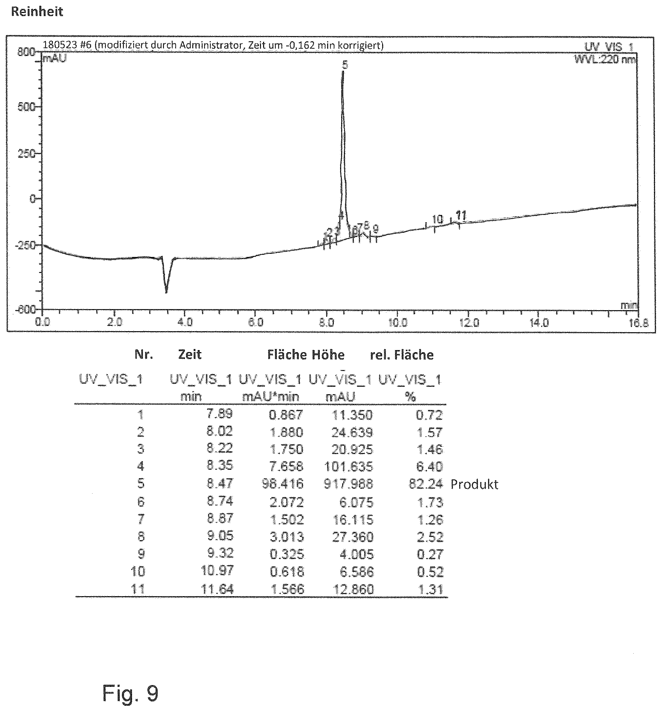

Figur 9 zeigt die Synthese des Acyl-Trägerproteins (ACP) mit der Sequenz VQAAIDYING-OH, hergestellt gemäß dem erfindungsgemäßen Verfahren mit einer Langzeitsynthese.

Es wurden die Schutzgruppen Fmoc-Q(Tmob)-OH und Fmoc-N(Tmob)-OH verwendet. Das synthetisierte Peptid ist aufgrund des stark hydrophoben Charakters grundsätzlich sehr schwer darstellbar. Dennoch konnte es mittels des erfindungsgemäßen Verfahrens mit einer Reinheit von 82 % dargestellt werden. Im Vergleich zu der in Figur 10 gezeigten Synthese des gleichen Peptids mit dem Verfahren Tetras nach dem Stand der Technik, bei dem nur eine Reinheit von 79 % erzielt werden konnte zeigt sich, dass das erfindungsgemäße Verfahren unter anderem eine Verbesserung der Ausbeuten erzielen kann. Zudem war die Synthesezeit des mittels dem erfindungsgemäßem Verfahren hergestellten Peptids in 2,5 h abgeschlossen, wohingegen das Vergleichsverfahren gemäß dem Stand der Technik 25h benötigte. Das erfindungsgemäße Verfahren ist somit zehnmal kürzer.

Die Verwendung des erfindungsgemäßen Verfahrens und der erfindungsgemäßen Vorrichtung führen vorteilhafterweise zu einer Reduktion der Synthesezeit auf maximal ein Zehntel der Synthesezeit für Verfahren nach dem Stand der Technik ohne Mikrowellenunterstützung. Es konnte gezeigt werden, dass dies keinesfalls mit einer Reduktion der Ausbeute zusammenläuft, vielmehr konnte bei einem direkten Vergleich mit einem Standardverfahren gezeigt werden, dass das erfindungsgemäße Verfahren, insbesondere bei Anwendung der erfindungsgemäßen Vorrichtung, eine höhere Reinheit des Zielpeptids erzeugte.

### Bezugszeichenliste

- 1: Synthesevorrichtung
- 2: Greifarm
- 3: Arbeitsfläche
- 4: Halterung
- 5: Syntheseplatte
- 6: Ventilblock
- 7: Absaugpumpe
- 8: Spülkamm
- 9: Reaktionskammern
- 10: Spülmittelzuführung
- 11: Synthesestift
- 12: Grundkörper, Hohlzylinder
- 13, 13a: Verschluss, Schraubverschluss, beweglicher Deckel mit Bajonettverschluss
- 13b: Arretierung Bajonettverschluss
- 14: Mundstück
- 15: Ventilnadel
- 16: Sperrventil
- 17: Kolbenstange
- 18: Kolben
- 19: Druckfeder
- 20: Synthesebaustein
- 21: Schutzgas
- 23: Auslauföffnung
- 25: permeables Material/Fritte
- 26: Festphase
- 27: Probenplatte
- 28: Membran
- 29: Dichtung
- 30: Greifarmaufnahme
- 31: Dosierzylinder
- 32: Auslassventil
- 33: Rückholfeder
- 33a: Befestigung für Rückholfeder
- 33b: Befestigung Rückholfeder, Schraubenmade
- 34: Spalt für Zylinderbefüllung
- 35: Überwurfmutter
- 36: Führung Dosierkanüle
- 37: Dosierkanüle

- 50: Ultraschallbad

- O: Vorquellen
- I: Binden einer am N-Terminus mit einer Schutzgruppe geschützten Aminosäure über einen C-Terminus der Aminosäure an ein festes Trägermaterial,
- II: Abspalten der Schutzgruppe
- III: Durchführen zumindest einer Peptidpropagation
- IV: Abbrechen der Reaktion durch Abspalten des Peptids vom Trägermaterial
- V: Waschen
- X: Ultraschalleinwirkung

## Patentansprüche

1. Verfahren zur Durchführung einer Festphasenpeptidsynthese umfassend die Schritte
a) Binden einer am N-Terminus mit einer Schutzgruppe geschützten Aminosäure über einen C-Terminus der Aminosäure an ein festes Trägermaterial;
b) Abspalten der Schutzgruppe;
c) Durchführen zumindest einer Peptidpropagation; und
d) Abbrechen der Reaktion durch Abspalten des Peptids vom Trägermaterial, wobei die Schritte a) bis d) in einem flüssigen Reaktionsmedium stattfinden und zumindest während einer der Schritte zumindest zeitweise Ultraschall mit einer Frequenz im Bereich von mehr als 100 bis 2000 kHz auf das Reaktionsmedium wirkt.

2. Verfahren nach Anspruch 1, **dadurch gekennzeichnet, dass** der Ultraschall mit einer Frequenz von mehr als 110 kHz auf das Reaktionsmedium wirkt.

3. Verfahren nach Anspruch 1 oder 2, **dadurch gekennzeichnet, dass** der Ultraschall mit einer Frequenz im Bereich von nicht mehr als 1000 kHz, vorzugsweise nicht mehr als 500 kHz auf das Reaktionsmedium wirkt.

4. Verfahren nach einem der vorhergehenden Ansprüche, **dadurch gekennzeichnet, dass** der Ultraschall über ein externes flüssiges Bad auf das Reaktionsmedium übertragen wird.

5. Verfahren nach einem der vorhergehenden Ansprüche, weiter umfassend einen nach Schritt b) stattfindenden Waschschritt W_{b}), einen nach Schritt c) stattfindenden Waschschritt W_{c}) und/oder einen nach dem Schritt d) stattfindenden Waschschritt W_{d}), wobei zumindest während einem dieser Schritte ebenfalls Ultraschall auf das Reaktionsmedium wirkt.

6. Verfahren nach einem der vorhergehenden Ansprüche, **dadurch gekennzeichnet, dass** die Aminosäure am N-Terminus durch eine basenlabile, insbesondere eine mittels sekundären Aminen abspaltbare Schutzgruppe, insbesondere Fluorenylmethoxycarbonyl (Fmoc), geschützt ist.

7. Verfahren nach einem der vorhergehenden Ansprüche, **dadurch gekennzeichnet, dass** die Aminosäure eine Schutzgruppe zum Schutz einer Seitenkette aufweist, insbesondere S-2,4,6-Trimethoxybenzyl (Tmob), Triphenylmethyl (Trt), tert-Butyl (tBu), tert-Butyloxycarbonyl (Boc), 2,2,4,6,7-Pentamethyldihydrobenzofuran-5-sulfonyl (Pbf).

8. Verfahren nach einem der vorhergehenden Ansprüche, **dadurch gekennzeichnet, dass** Ultraschall in genau einem Schritt, insbesondere in Schritt c), auf das Reaktionsmedium einwirkt.

9. Verfahren nach einem der vorhergehenden Ansprüche, **dadurch gekennzeichnet, dass** während aller Schritte a) bis d) und/oder W_{b}), W_{c}) und W_{d}) unterbrechungsfrei und/oder mit gleicher Frequenz Ultraschall auf das Reaktionsmedium einwirkt.

10. Verfahren nach einem der vorhergehenden Ansprüche, **dadurch gekennzeichnet, dass** wenn Ultraschall in mehreren Schritten auf das Reaktionsmedium einwirkt, die Ultraschallfrequenz zwischen den besagten Schritten, insbesondere zwischen den Reaktionsschritten a) bis d) und Waschschritten W_{b)} bis W_{-d}) variiert.

11. Verfahren nach einem der vorhergehenden Ansprüche, **dadurch gekennzeichnet, dass** die Frequenz während zumindest einem der Waschschritte W_{b)} bis W_{d}) mehr als 100 kHz, bevorzugt mehr als 110 kHz, beträgt.

12. Verfahren nach einem der vorhergehenden Ansprüche, **dadurch gekennzeichnet, dass** das Ultraschallbad auf einen Temperaturbereich von 20 bis 100 °C, vorzugsweise von 20 bis 70 °C, besonders bevorzugt von 40 bis 60 °C, geregelt wird.

13. Verfahren nach einem der vorhergehenden Ansprüche, **dadurch gekennzeichnet, dass** das Verfahren halb-/automatisiert und/oder parallel durchgeführt wird.

14. Verfahren nach einem der vorhergehenden Ansprüche, wobei Schritt d) einen Dosierschritt, einen Waschschritt W_{d)} und einen Filterschritt umfasst, und bei halbautomatisierter Durchführung, der Dosierschritt manuell und die weiteren Schritte automatisiert durchgeführt werden.

15. Automatisierte parallele Festphasenpeptidsynthese umfassend ein Verfahren nach einem der vorhergehenden Ansprüche.

16. Vorrichtung (1) zur Durchführung einer Festphasenpeptidsynthese eingerichtet zur Durchführung eines Verfahrens nach einem der vorhergehenden Ansprüche 4-15, aufweisend ein Mittel (4) zur Aufnahme eines Synthesegefäßes (5), insbesondere einer Syntheseplatte, eine Mehrzahl von Synthesezylindern, einen Reaktionskolben oder Reaktor mit zumindest einer Öffnung zum Einfüllen von Reaktandenmedien sowie ein eine Flüssigkeit aufweisendes Ultraschallbad (50), wobei das Synthesegefäß (5) derart im Ultraschallbad (50) anordenbar ist, dass das Synthesegefäß (5) bis zu einer minimalen Höhe mit der Flüssigkeit des Ultraschallbades (50) benetzt ist, wobei das Ultraschallbad höhenverstellbar eingerichtet ist, und wobei für einzelne Synthesebausteine jeweils separate Synthesestifte vorgesehen sind, die in einer Halterung für die Synthese bereitgestellt sind und aus dieser Halterung vom Greifarm der Vorrichtung (1) erfasst und aufgenommen werden, um eine dosierte Menge des Bausteins auf das in einer Reaktionskammer des Synthesegefäßes (5), insbesondere der Syntheseplatte, befindliche Trägermaterial abzugeben.

17. Vorrichtung nach Anspruch 16, **dadurch gekennzeichnet, dass** das Ultraschallbad temperierbar eingerichtet ist.

## Claims

1. A method for performing a solid-phase peptide synthesis comprising the steps of:
a) binding an amino acid protected at the N-terminus with a protective group via a C terminus of the amino acid to a solid carrier material;
b) cleaving off the protective group;
c) performing at least one peptide propagation; and
d) terminating the reaction by cleaving off the peptide from the carrier material,
wherein steps a) to d) take place in a liquid reaction medium and at least during one of the steps ultrasound with a frequency in the range from more than 100 to 2000 kHz acts at least temporarily on the reaction medium.

2. The method according to Claim 1, **characterized in that** the ultrasound acts on the reaction medium at a frequency of more than more than 110 kHz.

3. The method according to Claim 1 or 2, **characterized in that** the ultrasound acts on the reaction medium at a frequency in the range of no more than 1000 kHz and preferably of no more than 500 kHz.

4. The method according to any one of the preceding claims, **characterized in that** the ultrasound is transferred to the reaction medium via an external liquid bath.

5. The method according to any one of the preceding claims further comprising a washing step W_{b}) taking place after step b), a washing step W_{c}) taking place after step c) and/or a washing step W_{d}) taking place after step d), wherein ultrasound likewise acts on the reaction medium at least during one of said steps.

6. The method according to any one of the preceding claims, **characterized in that** the amino acid is protected at the N terminus by a protective group, in particular fluorenylmethoxycarbonyl (Fmoc), which is base-labile and is in particular cleavable by way of secondary amines.

7. The method according to any one of the preceding claims, **characterized in that** the amino acid has a protective group for protecting a side chain, in particular S-2,4,6-trimethoxybenzyl (Tmob), triphenylmethyl (Trt), tert.-butyl (tBu), tert-butyloxycarbonyl (Boc), 2,2,4,6,7-pentamethyldihydrobenzofuran-5-sulfonyl (Pbf).

8. The method according to any one of the preceding claims, **characterized in that** ultrasound acts on the reaction medium in precisely one step, in particular in step c).

9. The method according to any one of the preceding claims, **characterized in that** ultrasound acts on the reaction medium uninterruptedly and/or at the same frequency during all of steps a) to d) and/or W_{b}), W_{c}) and W_{d}).

10. The method according to any one of the preceding claims, **characterized in that**, if ultrasound acts on the reaction medium in a plurality of steps, the ultrasound frequency varies between said steps, in particular between reaction steps a) to d) and washing steps W_{b}) to W_{d}).

11. The method according to any one of the preceding claims, **characterized in that** the frequency during at least one of the washing steps W_{b}) to W_{d}) amounts to more than 100 kHz and preferably to more than 110 kHz.

12. The method according to any one of the preceding claims, **characterized in that** the ultrasound bath is controlled to a temperature range of 20 to 100°C, preferably of 20 to 70°C and particularly preferably of 40 to 60°C.

13. The method according to any one of the preceding claims, **characterized in that** the process is performed (semi-)automatically and/or in parallel.

14. The method according to any one of the preceding claims, wherein step d) comprises a dispensing step, a washing step W_{d}) and a filtering step, and, if performed semiautomatically, the dispensing step is performed manually and the further steps are performed automatically.

15. An automated parallel solid-phase peptide synthesis comprising a process according to any one of the preceding claims.

16. A device (1) for performing a solid-phase peptide synthesis set up for performing a process according to any one of the preceding Claims 4-15, having a means (4) for receiving a synthesis vessel (5), in particular a synthesis plate, a plurality of synthesis cylinders, a reaction flask or reactor with at least one opening for introduction of reactant media, and an ultrasound bath (50) holding a liquid, wherein the synthesis vessel (5) is arrangeable in the ultrasound bath (50) in such a way that the synthesis vessel (5) is wetted up to a minimum height by the liquid of the ultrasound bath (50), wherein the ultrasound bath is set up to be height-adjustable, and wherein a separate pen-type applicator is provided for each individual synthesis building block, which pen-type applicators are made available for synthesis in a holder and are gripped and picked up from this holder by the gripper arm of the device (1) in order to release a metered quantity of the building block onto the carrier material located in a reaction chamber of the synthesis vessel (5), in particular of the synthesis plate.

17. The device according to Claim 16, **characterized in that** the ultrasound bath is set up to be temperature-controllable.

## Revendications

1. Procédé d'exécution d'une synthèse de peptides en phase solide, comprenant les étapes suivantes :
a) liaison à un matériau support solide d'un acide aminé protégé par un groupe de protection à l'extrémité N terminale par une extrémité C terminale de l'acide aminé ;
b) séparation du groupe de protection ;
c) exécution d'au moins une propagation de peptide ; et
d) interruption de la réaction par séparation du peptide du matériau support,
les étapes a) à d) ayant lieu dans un milieu de réaction liquide, et des ultrasons à une fréquence supérieure à 100 kHz et allant jusqu'à 2000 kHz agissant sur le milieu de réaction au moins lors d'une des étapes, et au moins temporairement.

2. Procédé selon la revendication 1, **caractérisé en ce que** les ultrasons agissent à une fréquence supérieure à 110 kHz sur le milieu de réaction.

3. Procédé selon la revendication 1 ou la revendication 2, **caractérisé en ce que** les ultrasons agissent sur le milieu de réaction à une fréquence non supérieure à 1000 kHz, préférentiellement non supérieure à 500 kHz.

4. Procédé selon l'une quelconque des revendications précédentes, **caractérisé en ce que** les ultrasons sont transmis par un bain liquide externe au milieu de réaction.

5. Procédé selon l'une quelconque des revendications précédentes, comprenant en outre une étape de lavage W_{b}) consécutive à l'étape b), une étape de lavage W_{c}) consécutive à l'étape c) et/ou une étape de lavage W_{d}) consécutive à l'étape d), des ultrasons agissant également sur le milieu de réaction au moins pendant une de ces étapes.

6. Procédé selon l'une quelconque des revendications précédentes, **caractérisé en ce que** l'acide aminé est protégé à l'extrémité N terminale par un groupe de protection labile en présence d'une base, en particulier séparable au moyen d'amines secondaires, en particulier du fluorénylméthoxycarbonyle (Fmoc).

7. Procédé selon l'une quelconque des revendications précédentes, **caractérisé en ce que** l'acide aminé présente un groupe de protection d'une chaîne latérale, en particulier 5-2,4,6-triméthoxybenzyl (Tmob), triphénylméthyle (Trt), tert-butyle (tBu), tert-butyloxycarbonyle (Boc), 2,2,4,6,7- pentaméthyldihydrobenzofuran-5-sulfonyl (Pbf).

8. Procédé selon l'une quelconque des revendications précédentes, **caractérisé en ce que** des ultrasons agissent sur le milieu de réaction lors d'une seule étape, en particulier lors de l'étape c).

9. Procédé selon l'une quelconque des revendications précédentes, **caractérisé en ce que** des ultrasons agissent sans interruption et/ou à la même fréquence sur le milieu de réaction pendant toutes les étapes de a) à d) et/ou W_{b}), W_{c}) et W_{d}).

10. Procédé selon l'une quelconque des revendications précédentes, **caractérisé en ce que**, si des ultrasons agissent sur le milieu de réaction lors de plusieurs étapes, la fréquence ultrasonique varie entre lesdites étapes, en particulier entre les étapes de réaction a) à d) et les étapes de lavage W_{b}) à W_{d}).

11. Procédé selon l'une quelconque des revendications précédentes, **caractérisé en ce que** la fréquence est supérieure à 100 kHz, préférentiellement à 110 kHz pendant au moins une des étapes de lavage W_{b}) à W_{d}).

12. Procédé selon l'une quelconque des revendications précédentes, **caractérisé en ce que** le bain ultrasonique est réglé à une plage de température comprise entre 20 et 100 °C, préférentiellement entre 20 et 70 °C, tout particulièrement entre 40 et 60 °C.

13. Procédé selon l'une quelconque des revendications précédentes, **caractérisé en ce que** le procédé est exécuté de manière semi-automatisée et/ou parallèle.

14. Procédé selon l'une quelconque des revendications précédentes, où l'étape d) comprend une étape de dosage, une étape de lavage W_{d}) et une étape de filtrage, et où, en cas d'exécution semi-automatisée, l'étape de dosage est exécutée manuellement, et les autres étapes automatiquement.

15. Synthèse parallèle automatisée de peptides en phase solide, comprenant un procédé selon l'une quelconque des revendications précédentes.

16. Dispositif (1) pour l'exécution d'une synthèse de peptides en phase solide, prévu pour l'exécution d'un procédé selon l'une quelconque des revendications 4 à 15, comprenant un moyen (4) pour la réception d'un récipient de synthèse (5), en particulier d'une plaque de synthèse, une pluralité de cylindres de synthèse, un ballon à réaction ou un réacteur avec au moins une ouverture pour le versement de milieux réactifs, ainsi qu'un bain ultrasonique (5) contenant un liquide (50), le récipient de synthèse (5) pouvant être disposé dans le bain ultrasonique (50) de manière à mouiller le récipient de synthèse (5) jusqu'à une hauteur minimale avec le liquide du bain ultrasonique (50), le bain ultrasonique étant prévu réglable en hauteur, et des stylets de synthèse séparés étant respectivement prévus pour des éléments de synthèse particuliers, préparés dans un support pour la synthèse et saisis hors dudit support et recueillis par le bras préhenseur du dispositif (1), pour déposer une quantité dosée de l'élément sur le matériau support présenté dans une chambre de réaction du récipient de synthèse (5), en particulier de la plaque de synthèse.

17. Dispositif selon la revendication 16, **caractérisé en ce que** le bain ultrasonique est prévu de manière tempérable.
